(19)

Europäisches Patentamt

European Patent Office

Office européen des brevets

(11) **EP 4 732 894 A1**

(12) **EUROPEAN PATENT APPLICATION**

(43) Date of publication:
**29.04.2026 Bulletin 2026/18**

(21) Application number: **24208070.3**

(22) Date of filing: **22.10.2024**

(51) International Patent Classification (IPC):
**A61N 5/10** (2006.01)

(52) Cooperative Patent Classification (CPC):
**A61N 5/103**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers International AG 6312 Steinhausen (CH)**

(72) Inventor: **HAKALA, Mikko 05200 Rajamäki (FI)**

(74) Representative: **Mathisen & Macara LLP Charta House 30-38 Church Street Staines-upon-Thames TW18 4EP (GB)**

(54) **MULTIPLE PLAN EVALUATION FUNCTIONS IN RADIATION TREATMENT PLAN SEARCH AND SELECTION**

(57)    A radiotherapy treatment plan generation apparatus for searching for and selecting a radiotherapy treatment plan by an iterative process is provided. One or more first radiotherapy treatment plans are received (312). In a first iteration: one or more first plan evaluation functions are received (324); first costs (336) associated with each of the first radiotherapy treatment plans (312) are calculated using each of the first plan evaluation functions (312); a first visual arrangement (335) depicting an identity of each of said first radiotherapy treatment plans (312), an identity of each of said first plan evaluation functions (324) and first costs (336), is constructed, and the first visual arrangement (335) is displayed. Subsequent similar iterations are made until a plan is selected.

```
First iteration for plan selection

Inputted number of plans to evaluate: 4                        330

Uploaded plans:
     Plan 1: Optimised Gantry Angle IMRT
     Plan 2: Butterfly Optimised VMAT
     Plan 3: Full Arc VMAT
     Plan 4: Tangential IMRT

Inputted number of plan evaluation functions: 5

Uploaded plan evaluation functions:
     F1: Plan deliverability metrics
     F2: Fluence complexity metrics
     F3: Dosimetric scorecard functions
     F4: Plan accuracy metrics
     F5: Clinical goal based metrics

=====                                          334

Costs table:

            F1     F2     F3     F4     F5     Score
   Weight   40%    10%    30%    15%    5%
   Plan 1   2.1    1.3    4.1    1.2    1.0    2.43
   Plan 2   1.2    3.7    2.4    1.0    1.1    1.775
   Plan 3   2.7    1.0    1.9    3.1    4.4    2.435
   Plan 4   1.8    1.0    3.3    2.3    2.7    2.29

                                    336

Proceed to select plan y/n? n
```

FIG. 3D

EP 4 732 894 A1

**Description**

**TECHNICAL FIELD**

This invention relates to radiation therapy treatment plan evaluation and selection, and in particular, to methods and systems for a computer guided interactive and iterative process to evaluate multiple radiation treatment plans using multiple plan evaluation functions to assist in radiation treatment plan search and selection.

**BACKGROUND**

The use of energy to treat medical conditions comprises a known area of prior art endeavour. For example, radiation therapy comprises an important component of many treatment plans for reducing or eliminating unwanted tumours. Unfortunately, applied energy does not inherently discriminate between unwanted material and adjacent tissues, organs, or the like that are desired or even critical to continued survival of the patient (such adjacent tissues may be referred to as "organs at risk", OARs). As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform "machine" parameters during each of a plurality of sequential fields. For example, machine parameters of a radiotherapy system may include the angle of irradiation of the treatment beam, or the configuration of the leaves of a multileaf collimator.

When creating a radiotherapy treatment plan for external beam radiotherapy, such as photon or particle therapy, the planning process typically involves an optimisation step in which the machine parameters including the leaf modulation is optimised to produce an optimal energy deposition at target volumes to be treated, with the simultaneous aim of minimising harm to the surrounding tissue and sensitive organs of the patient.

Optimisation of radiotherapy treatment plans may involve minimising a cost function such as a quadratic cost function defined by optimisation objectives. If the parameters that define the cost function are altered at any point of the planning process, it essentially means that the plan is optimised with respect to a slightly different cost function. Moreover, cost functions other than quadratic cost functions can be utilised by treatment planning software.

On the other hand, the creation of a treatment plan can be seen as an attempt to optimise any kind of a plan quality function, which can be again defined in various ways depending on a physician's preferences and a particular patient's anatomy. Plan quality functions may depend on clinical goals and may use scorecards. Plan quality functions may or may not include non-dosimetric goals such as plan complexity or treatment time. Fully patient-specific plan quality functions may be generated for individualised treatment.

Thus, it is clear that there can be multiple plan evaluation functions that all describe the "goodness" of a plan from different (and partly overlapping) perspectives. In the present disclosure, such functions are referred to generally as "plan evaluation functions", (i.e., functions that generate metrics that reflect the quality of a treatment plan). As mentioned above, there are many different types of plan evaluation functions, such as different cost functions of optimisers and dosimetrically and otherwise motivated plan quality functions.

The problem addressed by the present disclosure is that a clinic or a planner may have access to multiple plan evaluation functions, but there is no definite method for easily evaluating and selecting a treatment plan when there are multiple plan evaluation functions available to assess the quality of the multiple treatment plans. There is a need to easily identify the most appropriate plan even when there are numerous plan evaluation functions available to evaluate each plan.

**SUMMARY OF THE INVENTION**

In accordance with the invention, there is provided a radiotherapy treatment plan generation apparatus for searching for and selecting a radiotherapy treatment plan by an iterative process, as defined by claim 1.

Optional features are defined by the dependent claims.

In accordance with the invention, there is provided a radiotherapy treatment plan generation apparatus for searching for and selecting a radiotherapy treatment plan by an iterative process, the apparatus configured to receive one or more first radiotherapy treatment plans and further configured to:

in a first iteration:

receive one or more first plan evaluation functions;
calculate first costs associated with each of the first radiotherapy treatment plans using each of the first plan evaluation functions;
construct a first visual arrangement depicting an identity of each of said first radiotherapy treatment plans, an identity of each of said first plan evaluation functions and first costs; and
display said first visual arrangement;

and in one or more subsequent iterations:

receive one or more subsequent plan evaluation functions;
calculate subsequent costs associated with each of the first radiotherapy treatment plans using each of the subsequent plan evaluation functions;
construct a subsequent visual arrangement depicting an identity of each of said first radiotherapy treatment plans, an identity of each of said subsequent plan evaluation functions and subsequent costs;
display said subsequent visual arrangement;
stop the iterative process upon receiving a selection of a final radiotherapy treatment plan; and
outputting said selected final radiotherapy treatment plan.

Thus, the results of iteratively evaluating multiple radiotherapy treatment plans using multiple plan evaluation functions are displayed at each iteration so that a human user may easily comprehend the evaluation results at the end of each iteration. Advantageously, this provides a computer-guided search of radiotherapy treatment plans to enable the final selection of a treatment plan by a human user or computer.

This is useful, for example, when different plans are calculated to be the "best" plan when the plans are evaluated using different plan evaluation functions.

Each subsequent iteration may also optionally receive one or more subsequent radiotherapy treatment plans for calculation of subsequent costs using the subsequent plan evaluation functions, for display in the subsequent visual arrangement.

A computer console may provide one or more prompts for the human user to input the first (and optional subsequent) radiotherapy treatment plans, and the first and subsequent plan evaluation functions. The first (and optional) subsequent radiotherapy treatment plans, and the first and subsequent plan evaluation functions, may then be received by means of human user input into the console.

The first and subsequent costs may be calculated, and the first and subsequent visual arrangements may be displayed, upon a corresponding human user input to proceed with the calculating of the costs and the displaying of the visual arrangements. The input to proceed with the costs calculation and/or proceed with the visual arrangement display may take place after the human user has inputted the first or subsequent plan evaluation functions, and after the human user has inputted the first (or optional subsequent) radiotherapy treatment plans.

At the end of each iteration, the console may allow the human user to elect to proceed to the next iteration, or to select a treatment plan.

*Changes from iteration to iteration*

The radiotherapy treatment plans in a subsequent iteration may be the same as the radiotherapy treatment plans of an immediately preceding iteration. For example, the same treatment plans may be evaluated using different plan evaluation functions.

The radiotherapy treatment plans in a subsequent iteration may be the same as the radiotherapy treatment plans of an immediately preceding iteration, but may also include one or more additional, radiotherapy treatment plans.

The radiotherapy treatment plans in a subsequent iteration may have some plans in common with the radiotherapy treatment plans of an immediately preceding iteration, but some plans of the immediately preceding iteration may be removed in the subsequent iteration. In this case, optionally, the plans of the subsequent iteration may include one or more additional radiotherapy treatment plans.

The radiotherapy treatment plans in a subsequent iteration may not have any radiotherapy treatment plans in common with those of an immediately preceding iteration.

The plan evaluation functions in a subsequent iteration may be the same as the plan evaluation functions of an immediately preceding iteration. For example, different radiotherapy treatment plans may be evaluated using the same plan evaluation functions.

The plan evaluation functions in a subsequent iteration may be the same as the plan evaluation functions of an immediately preceding iteration, but may also include one or more additional, plan evaluation functions.

The plan evaluation functions in a subsequent iteration may have some plan evaluation functions in common with the plan evaluation functions of an immediately preceding iteration, but some plan evaluation functions may be removed in the subsequent iteration. In this case, optionally, the plan evaluation functions of the subsequent iteration may include one or more additional plan evaluation functions.

The plan evaluation functions of a subsequent iteration may not have any plan evaluation functions in common with those of an immediately preceding iteration.

*Plan evaluation function types*

Optionally, the first plan evaluation functions and/or the subsequent plan evaluation functions comprise two or more plan evaluation functions that relate to different types of plan evaluation functions, wherein said types of plan evaluation functions include two or more of:

(i) plan quality functions of automated optimisers;
(ii) dosimetric scorecard functions;
(iii) clinical goal based metrics;
(iv) fluence complexity metrics;
(v) plan deliverability metrics;
(vi) plan accuracy metrics;
(vii) dose optimiser based objective functions; and
(viii) individualised plan quality functions.

The above list of plan evaluation functions is merely exemplary and is not intended to be limiting. Any type of plan evaluation function may be used with the present embodiments.

A plan evaluation function may comprise a mathematical equation or an algorithm that provides a numerical quantification of the quality of a radiotherapy treatment plan. The results of each type of plan evaluation function may comprise a real number of any value.

Each plan evaluation function type may provide a cost associated with each radiotherapy treatment plan. Different radiotherapy treatment plans may have the best cost when assessed by different plan evaluation functions.

The plan evaluation functions of each iteration may be selected by means of e.g. a drop-down menu of the computer console.

Optionally, the first plan evaluation functions and/or the subsequent plan evaluation functions comprise different plan evaluation functions of the same type.

In particular, two of the plan evaluation functions may be of the same type but comprise different parameters or term coefficients. For example, two plan evaluation functions may both comprise clinical goal based metrics, but have different weights for the different goals.

*Radiotherapy treatment plan types*

Optionally, the first radiotherapy treatment plans comprise two or more radiotherapy treatment plans that relate to different types of radiotherapy, wherein said types of radiotherapy include two or more of:

(i) Templatised Intensity Modulated Radiotherapy;
(ii) Tangential Intensity Modulated Radiotherapy;
(iii) Optimised Gantry Angle Intensity Modulated Radiotherapy:
(iv) Manually Set Gantry Angles Intensity Modulated Radiotherapy;
(v) Full Arc Volumetric Arc Therapy;
(vi) Butterfly Templatised Volumetric Arc Therapy;
(vii) Butterfly Optimised Volumetric Arc Therapy;
(viii) Butterfly Manually Set Volumetric Arc Therapy;
(ix) Partial Arc Templatised Volumetric Arc Therapy;
(x) Partial Arc Optimised Volumetric Arc Therapy; and
(xi) Partial Arc Manually Set Volumetric Arc Therapy.

The above list of radiotherapy treatment plan types is merely exemplary and is not intended to be limiting. Any type of radiotherapy treatment plan may be used with the present embodiments. For example, each of the above eleven types of radiotherapy treatment plans may be optimised using either a fixed-angle for the collimator, or an optimisable collimator angle. This would provide another eleven types of treatment plans. Many other aspects of the treatment may be changed to provide further types of radiotherapy treatment plans.

When evaluated by different plan evaluation functions, a radiotherapy treatment plan of a radiotherapy treatment plan type may have different costs. Different radiotherapy treatment plan types may be "best" in dependence on the plan evaluation function used to evaluate the radiotherapy treatment plans.

Radiotherapy treatment plans may be generated and optimised using inverse planning and cost function minimisation, as is well known in the art. Radiotherapy treatment plans of different types may be generated and optimised using different radiotherapy treatment planning algorithms or different cost functions.

The radiotherapy treatment plans of an iteration may be selected by means of e.g. a drop-down menu of the computer console.

Optionally, the first radiotherapy treatment plans comprise different radiotherapy treatment plans of the same type.

In particular, two of the radiotherapy treatment plans may be of the same type but comprise different parameters such as different beam angles or different beam intensities. For example, two of the radiotherapy treatment plans may both comprise Partial Arc Optimised Volumetric Arc therapy, but may specify different beam-on arc lengths.

*Weighting costs*

Optionally, the apparatus is further configured to weight one or more of the first and/or subsequent costs using respective weights associated with each respective first and/or subsequent plan evaluation function.

Advantageously, by providing the option of weighting the costs using weights associated with each plan evaluation function, it is possible to emphasise different plan evaluation functions in dependence on the relative importance of each plan evaluation function. This allows more effective comparison of the different radiotherapy treatment plans as evaluated by the different, weighted, plan evaluation functions.

Weighting the plan evaluation functions also allows a weighted sum of costs to be calculated for each radiotherapy treatment plan, wherein the weighted sum reflects the overall quality of a plan whilst taking into account the different importances of the different plan evaluation functions. That is, the sum of all the weighted costs comprises a "total score" that reflects the combined value of a radiotherapy treatment plan as evaluated by plan evaluation functions of varying importance. This allows for more effective comparison of plans.

Applying a weight may comprise multiplying the weight value with the cost associated with a radiotherapy treatment plan.

The weights of the plan evaluation functions may each comprise a percentage or a fraction. Where the weights comprise a percentage, the sum of all the weights may be 100%. Where the weights comprise a fraction, the sum of all the weights may be 1.

In some examples, the plan evaluation functions may all have the same importance, in which case the weight assigned to each plan evaluation function may be the same. For example, where there are $N$ plan evaluation functions, each plan evaluation function may be assigned a weight of $1/N$ (where the weight is a fraction) or each plan evaluation function may be assigned a weight of $100/N$ (where the weight is a percentage).

Each weight may be displayed along with its corresponding plan evaluation function in the first and/or subsequent visual arrangements. For example, where the first and/or subsequent visual arrangements comprise a table, the plan evaluation functions may be listed in the top row of the table and the corresponding weight of each plan evaluation function may be displayed in the row below the top row of the table.

The weighted sum of all costs associated with a radiotherapy treatment plan may also be included in the visual arrangement. For example, where the first and/or subsequent visual arrangements comprise a table, each radiotherapy treatment plan may be assigned to a particular row and the weighted sum of costs associated with each radiotherapy treatment plan may be displayed in the corresponding row, e.g. in the rightmost column.

The computer console may allow a human user to input the weights associated with each plan evaluation function. The computer console may allow a human user to revise the weights associated with each plan evaluation function. The computer console may allow the weights to be inputted at the start of every iteration or at the start of one or more iterations.

Alternatively, in one or more iterations, the console may allow a human user to specify that all plan evaluation functions are to have the same importance. When all plan evaluation functions are to have the same importance in a particular iteration, equal weights, as described above, are assigned to each plan evaluation function.

In some cases, the only difference between any two iterations may be the weights associated with each plan evaluation function. For example, a subsequent iteration may relate to the same radiotherapy treatment plans and the same plan evaluation functions as a previous iteration, but may utilise different weights associated with each plan evaluation function.

*Normalising costs*

Optionally, the apparatus is further configured to determine a plurality of normalised costs for each of the first radiotherapy treatment plans by dividing each of the first and/or subsequent costs of each first radiotherapy treatment plan by a minimum cost out of all the costs for the respective first radiotherapy treatment plan.

Normalising the costs in this way provides a set of costs for each radiotherapy treatment plan that are scaled to have 1.0 as the lowest normalised cost, with no upper limit to the normalised cost. Thus, normalising causes every plan to have a minimum normalised cost of 1.0. Advantageously, normalising the costs of each radiotherapy treatment plan allows easier comparison of the plans because the costs are all defined in the same scale.

As the lowest normalised cost of any treatment plan is always 1.0, the one or more plans with a normalised cost of 1.0 for a particular plan evaluation function are the "best" plans when the plans are evaluated with that particular plan evaluation function. Also, the plan evaluation function that provides a normalised cost of 1.0 for a particular radiotherapy treatment plan indicates the plan evaluation function under which the particular radiotherapy treatment plan performs best (or joint best, if more than one plan evaluation function provides a normalised cost of 1.0 for a particular radiotherapy treatment plan).

The normalised costs of the plans as evaluated by the plan evaluation functions may be displayed in the first or subsequent visual arrangements. For example, the normalised costs may be displayed in addition to, or instead of, the non-normalised costs. For example, where the first and/or subsequent visual arrangements comprise a table, each row may relate to a particular radiotherapy treatment plan. An identity of the particular plan and the normalised costs associated with the radiotherapy treatment plan may be included in each row of the table.

In the normalisation process, as each cost of a plan as evaluated by each plan evaluation function is divided by the minimum cost of a plan, each row of the table will have at least one cell with a normalised cost of 1.0.

The console may allow a human user to specify whether or not to calculate normalised costs, whether or not the visual arrangements are to be displayed with normalised costs, and/or whether or not the visual arrangements are to be displayed with non-normalised costs.

*Both weighting and normalising costs*

Optionally, the apparatus is further configured to weight one or more of the plurality of normalised costs using respective weights associated with each respective first and/or subsequent plan evaluation function.

That is, in addition to or instead of weighting the costs or normalising the costs, the apparatus may provide for both weighting and normalising of the costs. This also allows for the calculation of a weighted sum of normalised costs for each plan.

Weighting and normalising the costs may be performed in any order. For example, first, each cost may be multiplied by the weight associated with the corresponding plan evaluation function, and then the weighted cost may be normalised by dividing by the lowest weighted cost. Alternatively, first, each cost may be normalised by dividing by the lowest cost, and then the normalised costs may be multiplied by the weight associated with the corresponding plan evaluation function. Both these alternatives result in the same sets of weighted normalised costs, the sets comprising one set of weighted normalised costs per radiotherapy treatment plan.

That is, weighted normalised costs may be determined by both (i) multiplying each cost with a weight associated with a plan evaluation function and (ii) dividing each cost by a minimum cost for a radiotherapy treatment plan.

The plan with the lowest sum of weighted normalised costs may be considered to be the (possibly joint) "best" plan. This "best" plan may then be selected and applied to a patient. Alternatively, it is possible that either the human user or the computer may determine that the "best" plan is not the plan with the lowest weighted sum of normalised costs, but rather may define the "best" plan in terms of alternative criteria. For example, even if a plan has the lowest overall weighted sum of normalised costs, it may have the highest costs for a majority of the plan evaluation functions. This may lead to a treatment plan having a score greater than the lowest overall weighted sum of normalised costs, being selected by the human user or the computer.

The console may allow the human user to specify whether or not to calculate and/or display in the visual arrangements, the weighted normalised costs associated with each radiotherapy treatment plan.

The display of weighted normalised costs and the display of a sum of weighted normalised costs allows for easier comparison of treatment plans.

*Ranges of costs across weights*

Optionally, the apparatus is further configured to vary weights associated with the first and/or subsequent plan evaluation functions and re-calculate the weighted costs and/or weighted normalised costs of the first radiotherapy treatment plans using the varying weights.

Advantageously, by varying the weights of the plan evaluation functions, the relative importance of the different plan evaluation functions may be varied at the discretion of the human user. The console may allow the human user to request a change to the weights, and the console may also allow the human user to then subsequently input new weight values.

After a change in the weights, the weighted costs or the weighted normalised costs may be recalculated. The sum of the weighted costs or the sum of the weighted normalised costs may also be recalculated. The visual arrangement may then be updated with the recalculated weighted costs, recalculated weighted normalised costs, recalculated sum of weighted costs and/or recalculated sum of weighted normalised costs.

In an example, the only difference between successive iterations may be the values of the weights. That is, the plans and evaluation functions of different iterations may be identical, but the weights of the plan evaluation functions may be different in these iterations. This allows the human user to assess each plan using the same plan evaluation functions but wherein each plan evaluation function has varying importance while all other variables remain constant.

Optionally, the apparatus is further configured to calculate, and optionally display, a range of weighted costs corresponding to varying weights associated with the first and/or subsequent plan evaluation functions.

By varying the weights associated with a plan evaluation function, a number of different costs may be generated for any particular radiotherapy treatment plan using a single plan evaluation function. The lowest weighted cost and the highest weighted cost associated with each radiotherapy treatment plan and plan evaluation function constitutes a range of costs associated with the plan and evaluation function, wherein the range arises by varying the weights while keeping all other variables constant.

The console may provide the human user with the option of calculating and displaying the range of weighted costs corresponding to varying weights of each plan evaluation function, while keeping all other variables constant.

The range of weighted costs may further be normalised with the lowest weighted cost associated with a treatment plan.

The range of weighted costs or range of weighted normalised costs may be displayed in the visual arrangement. For example, where the visual arrangement comprises a table, each row may relate to a particular radiotherapy treatment plan and the cells in each row may show each range of weighted costs and/or each range of normalised weighted costs as calculated by the plan evaluation functions with varying weights.

Advantageously, the display in the visual arrangements of a range of costs for each plan arising due to varying weights, assists the human user in assessing and comparing the plans.

*Ranges of costs across plan evaluation functions*

Optionally, the apparatus is further configured to calculate, and optionally display, a range of costs corresponding to the different first and/or subsequent plan evaluation functions.

As described, a particular radiotherapy treatment plan may be evaluated using multiple plan evaluation functions. The lowest cost provided by the multiple plan evaluation functions and the highest cost provided by the multiple plan evaluation functions constitutes a range of costs associated with the particular radiotherapy treatment plan.

The range of costs associated with the varying plan evaluation functions may be weighted with a weight associated with the plan evaluation functions. The range of costs associated with the varying plan evaluation functions may be normalised with the corresponding lowest cost. The range of costs associated with the varying plan evaluation functions may be both weighted and normalised.

The console may provide the human user with the option of calculating and/or displaying the range of costs, the range of weighted costs and/or the range of weighted normalised costs corresponding to varying the plan evaluation function used to evaluate the radiotherapy treatment plans.

The range of costs, range of weighted costs and/or range of weighted normalised costs associated with varying plan evaluation functions may be displayed in the visual arrangement. For example, where the visual arrangement comprises a table, each row may relate to a radiotherapy treatment plan and the rightmost column of a respective row may provide the range of costs associated with the varying respective plan evaluation functions.

Advantageously, the display of a range of costs associated with varying plan evaluation functions in the visual arrangements assists the human user in assessing and comparing the plans.

*Ranges of costs across plans*

Optionally, the apparatus is further configured to calculate, and optionally display, a range of costs corresponding to the different radiotherapy treatment plans.

As described, multiple radiotherapy treatment plans may be evaluated using any one plan evaluation function. The treatment plans may comprise a treatment plan with the lowest cost as calculated by a particular plan quality function and a treatment plan with the highest cost as calculated by the particular plan quality function. This lowest cost and highest cost constitute a range of costs associated with the different radiotherapy treatment plans, whilst keeping all other variables constant.

The range of costs associated with the varying treatment plans may be weighted with a weight associated with the plan evaluation functions. The range of costs associated with the varying treatment plans may be normalised with the corresponding lowest cost. The range of costs associated the varying plans may be both weighted and normalised.

The console may provide the human user with the option of calculating and/or displaying the range of costs corresponding to varying the radiotherapy treatment plans while keeping all variables, including the plan evaluation function, constant.

The range of costs, range of weighted costs and/or range of weighted normalised costs associated with varying treatment plans may be displayed in the visual arrangement. For example, where the visual arrangement comprises a table, each column may relate to a plan evaluation function and the bottom row of a respective column may provide the range of costs associated with the varying treatment plans for any one particular plan evaluation function.

Advantageously, the display of a range of costs associated with varying treatment plans in the visual arrangements assists the human user in assessing and comparing the plans.

*Total scores*

Optionally, the apparatus is further configured to calculate, and optionally display, a total score for each of the first radiotherapy treatment plans comprising one or more of: a sum of costs, a sum of weighted costs, a sum of normalised costs and a sum of weighted normalised costs.

Optionally, the apparatus is configured to calculate and optionally display: one or more ranges of total scores for each of the radiotherapy treatment plans, the ranges comprising one or more of: a range of sums of costs, a range of sums of weighted costs, a range of sums of normalised costs and a range of sums of weighted normalised costs, wherein each range may correspond to varying weights, varying plan evaluation functions, or varying radiotherapy treatment plans.

The console may allow the user to request the calculation and display of the total scores and/or the ranges of total scores. The total scores and/or the ranges of total scores may then be included in the first and/or subsequent visual arrangements.

Advantageously, by displaying the total scores or ranges of total scores in the visual arrangements, a human user is assisted in comparing the treatment plans and a computer-guided process is provided by means of which a suitable treatment plan is selected.

*Visual arrangements*

Optionally, the apparatus is further configured to include in the first visual arrangement or in the subsequent visual arrangement, one or more of:

> (i) weighted first and/or subsequent costs for the first radiotherapy treatment plans,
> (ii) normalised first and/or subsequent costs for the first radiotherapy treatment plans,
> (iii) weighted normalised first and/or subsequent costs for the first radiotherapy treatment plans, and
> (iv) a total score for the first radiotherapy treatment plans,

as determined using each of the first and/or subsequent plan quality functions and corresponding weights.

A visual arrangement may display any combination of one or more of: identity of radiotherapy treatment plans, identity of plan evaluation functions, costs, weighted costs, normalised costs, weighted normalised costs, total score, ranges of costs, ranges of weighted costs, ranges of normalised costs, ranges of weighted normalised costs and ranges of total scores. The ranges may relate to varying weights, varying plan evaluation functions, or varying radiotherapy treatment plans.

The console may allow the user to specify what information the visual arrangements should provide. The information provided assists the human user in comparing treatment plans easily and effectively. Thus, the interaction with the console provides an ongoing computer-assisted search, comparison and selection of treatment plans.

*Subsequent treatment plans*

Optionally, the apparatus is further configured to, in the one or more subsequent iterations:

> receive one or more subsequent radiotherapy treatment plans;
> calculate subsequent costs associated with each of the subsequent radiotherapy treatment plans using each of the first and/or subsequent plan evaluation functions;
> and
> construct the subsequent visual arrangement depicting said subsequent radiotherapy treatment plans, first and/or subsequent plan evaluation functions and subsequent costs.

That is, in addition to receiving subsequent plan evaluation functions in the subsequent iterations, optionally, subsequent radiotherapy treatment plans may also be received. This increases the universe of radiotherapy treatment plans that are being displayed, compared and selected in the ongoing computer assisted search and selection of treatment plans.

The console may provide the user with the option of inputting new treatment plans at every iteration. The treatment plans may be pre-generated or generated during the present plan search and selection process, using, for example, traditional inverse planning and/or objective function optimisation. The new treatment plans may be of any of the types listed above.

*Plan selection*

Optionally, the apparatus is further configured to select the final radiotherapy treatment plan wholly or partially by a processor.

Optionally, the processor is configured to select the final radiotherapy treatment plan in dependence on one or more of: (i) respective costs, (ii) respective weighted costs, (iii) respective normalised costs, (iv) respective weighted normalised costs, and (v) respective total score.

The processor may utilise specialised software to compare the respective costs, weighted costs, normalised costs, weighted normalised costs, and total scores. The processor may, for example, select the plan with the lowest cost, lowest weighted cost, lowest normalised cost, lowest normalised weighted cost, or lowest total score.

In an example, the final plan may not be selected based on simple rules such as "lowest cost" or "lowest total score". Rather, more complex rules may be used in the plan selection. For example, a trade-off may occur where a plan has a very low cost when evaluated by almost all the evaluation functions, but still has a very high total score due to the remaining evaluation functions outputting a high cost. In this case, a plan with a total score higher than a total score of another plan may still be selected because a majority of the plan evaluation functions output a low cost for the plan.

The above plan selection rules are merely examples, and any number of rules for plan selection may be used.

The processor may be configured to select the final radiotherapy treatment plan using artificial intelligence, and for example, by means of a neural network. For example, the neural network may be trained by backpropagation to develop a set of neuron connection weights that can output the most appropriate treatment plan given a set of costs associated with the radiotherapy treatment plans, as calculated by the plan evaluation functions.

The console may provide the human user with the option of manual selection or the option of automatic selection. In the case of manual selection, the human user, with the assistance of the information displayed in the respective visual arrangements described above, may easily and effectively compare the plans to provide a selected treatment plan. In the case of automatic selection, the computer may compare the costs of all the treatment plans provided in all the iterations of the search process, and select a plan based on the rules described above.

*Cost vs. utility*

Instead of calculating and displaying costs, the processor may calculate and display utility. Whereas lower cost plans are considered superior to higher cost plans, higher utility plans are considered superior to lower utility plans. In this case, the processor may select the plan with the highest utility, highest weighted utility, highest normalised utility, highest normalised weighted utility, or highest total utility score. In general, throughout the present disclosure, where the cost of a plan is to be determined, this may instead involve determining the utility of the plan. The concepts of cost and utility are inversely related to each other and may be used in complementarily.

*Means for implementing functions*

In accordance with a further aspect of the invention, there is provided means for searching for and selecting a radiotherapy treatment plan by an iterative process, comprising:
means to receive one or more first radiotherapy treatment plans; and

   in a first iteration:

      means to receive one or more first plan evaluation functions;
      means to calculate first costs associated with each of the first radiotherapy treatment plans using each of the first plan evaluation functions;
      means to construct a first visual arrangement depicting an identity of each of said first radiotherapy treatment plans, an identity of each of said first plan evaluation functions and first costs; and
      means to display said first visual arrangement;

and in one or more subsequent iterations:

> means to receive one or more subsequent plan evaluation functions;
> means to calculate subsequent costs associated with each of the first radiotherapy treatment plans using each of the subsequent plan evaluation functions;
> means to construct a subsequent visual arrangement depicting an identity of each of said first radiotherapy treatment plans, an identity of each of said subsequent plan evaluation functions and subsequent costs;
> means to display said subsequent visual arrangement;
> means to stop the iterative process upon receiving a selection of a final radiotherapy treatment plan; and
> means to output said selected final radiotherapy treatment plan.

The means to receive the one or more first radiotherapy treatment plans, the means to receive the one or more first plan evaluation functions and the means to receive the one or more subsequent plan evaluation functions may comprise a console displaying a graphical user interface that allows a human user to input the relevant information. Computer systems that may implement a suitable console or graphical user interface are discussed in detail below.

The means to calculate costs, the means to construct visual arrangements, the means to stop the iterative process and the means to output said selected final radiotherapy treatment plan may comprise a computer processor as discussed in detail below.

The means to display the visual arrangements may comprise a computer monitor, as discussed in detail below.

## BRIEF DESCRIPTION OF THE DRAWINGS

For a better understanding of the present disclosure, embodiments will now be described by way of example with reference to the accompanying drawings, in which:

> FIG. 1 is an illustration of a typical radiotherapy treatment system;
> FIG. 2 is a side view of a radiotherapy treatment system in use;
> FIGS. 3A-3J are exemplary console screenshots of a first iteration, second iteration and a final (eighth) iteration of the computer-guided interactive process to evaluate multiple treatment plans using multiple plan evaluation functions;
> FIGS. 4A and 4B show a flowchart of a method to evaluate multiple treatment plans using multiple plan evaluation functions, in a computer-guided ongoing interactive process;
> FIG. 5 shows a typical computer system upon which the present method may be implemented.

## DETAILED DESCRIPTION

### Treatment system

In general, radiation therapy consists of the use of ionising radiation to treat living tissue, usually tumours. There are many different types of ionising radiation used in radiation therapy, including high energy x-rays, electron beams, and proton beams. The process of administering the radiation to a patient can be somewhat generalised regardless of the type of radiation used. External beam therapy (EBT), also called external radiation therapy, is a method for delivering a beam or several beams of e.g. high-energy x-rays to a patient's tumour. Beams are generated outside the patient (usually by a linear accelerator) and are targeted at the tumour site.

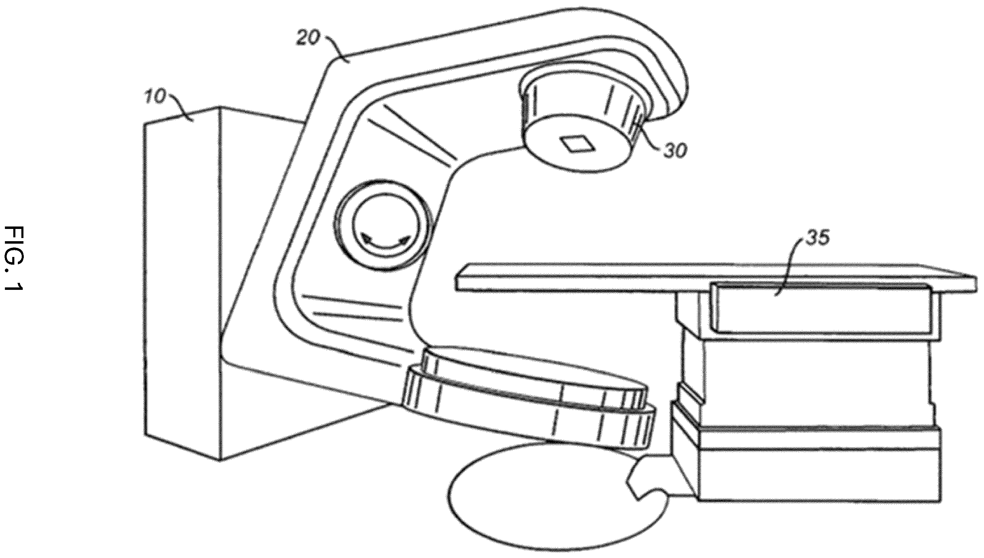

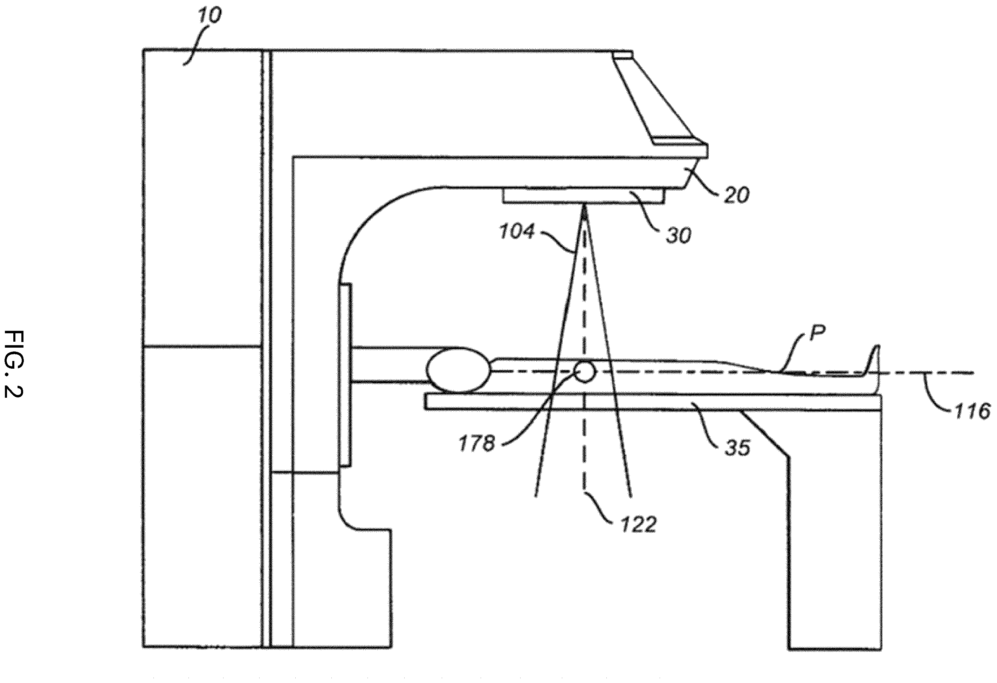

FIGS. 1 and 2 depict a radiation treatment system of the type that may be used in accordance with some embodiments. Referring to FIG. 1, a perspective view of a radiation treatment system (in this case a linear accelerator) is shown. Typically, such a system is capable of generating either a particle (e.g. electron) beam or a photon (e.g. x-ray) beam for use in the radiotherapy treatment of patients on a treatment couch 35. Some radiation treatment systems are capable of generating heavy ion particles such as protons. For purposes of the present discussion, only x-ray irradiation will be discussed. However, it will be appreciated by those skilled in the art that the same principles apply to other (e.g. particle beam) systems.

Stand 10 supports a rotatable gantry 20 with a treatment head 30. Next to stand 10 there is arranged a control unit (not shown) that includes control circuitry for controlling the different modes of operation of the accelerator. A high voltage source is provided within the stand or in the gantry, to supply voltage to an electron gun (not shown) positioned on an accelerator guide located in the gantry 20. Electrons are emitted from the electron gun into the guide (not shown) where they are accelerated. A source supplies RF (microwave) power for the generation of an electric field within the waveguide. The electrons emitted from the electron gun are accelerated in the waveguide by the electric field, and exit the waveguide as a high energy electron beam, typically at megavoltage energies. The electron beam then strikes a suitable metal target, emitting high energy x-rays in the forward direction.

Referring now to FIG. 2, a somewhat more detailed side view of a radiation treatment system is shown. A patient P is shown lying on the treatment couch 35. X-rays formed as described above are emitted from the target in the treatment head 30 in a divergent beam 104. Typically, a patient plane 116, which is perpendicular to the page in FIG. 2, is positioned about one meter from the x-ray source or target, and the axis of the gantry 20 is located on the plane 116, such that the distance between the target and the isocentre 178 remains constant when the gantry 20 is rotated. The isocentre 178 is at the intersection between the patient plane 116 and the central axis 122 of the beam 104. A treatment volume to be irradiated is located about the isocentre 178.

Although the above discussion relates to external beam therapy, it should be understood that the present invention is not so limited, and may, for example, be used with other types of radiation therapy such as brachytherapy.

Multiple plan evaluation functions in treatment plan selection

At the start of the treatment plan search and selection process according to the present disclosure, a plurality of treatment plans are generated for a particular patient. Each treatment plan may be of the same type, or there may be two or more treatment plans each being of a different treatment plan type. The treatment plans may be generated by a human planner or an automated planning system.

Further, there may be $N$ different plan evaluation functions that may be used to evaluate each of the different treatment plans. Each plan evaluation function may be of the same type, or there may be two or more plan evaluation functions each being of a different plan evaluation function type.

The plan evaluation functions may be referred to generally by the term $F\_n$, where $n = 1...N$. Plan evaluation function F_1 may be of a particular type, such as a dosimetric scorecard function. Plan evaluation function $F\_2$ may be of a different type, such as a function that evaluates the complexity of a treatment plan. Plan evaluation function $F\_3$ may be a fluence complexity metric, and so on.

Each plan evaluation function may be applied to each of the treatment plans. Each treatment plan has a plurality of associated costs, each cost reflecting the cost of the treatment plan as evaluated by a different plan evaluation function. In general, it is difficult or impossible to generate a single treatment plan wherein all the costs for the single treatment plan (as determined by all the plan evaluation functions F_1, F_2, F_3, ..., F_N) are the lowest in relation to the respective costs of all the other treatment plans.

That is, a first plan may achieve a lower cost than a second plan when the plans are evaluated using a first plan evaluation function, but the second plan may achieve a lower cost than the first plan when the plans are evaluated using a second plan evaluation function.

Thus, selecting a most appropriate plan from a plurality of treatment plans is not trivial, as some plans are superior when assessed by a particular plan evaluation function, while other plans may be superior when assessed by another plan evaluation function.

The present solution to treatment plan selection involves an iterative process in which in a first iteration, the costs of multiple treatment plans as evaluated by multiple plan evaluation functions are displayed in a visual arrangement in a display. In subsequent iterations, subsequent plan evaluation functions (and optionally, subsequent treatment plans) may be received, and the costs of the treatment plans as evaluated by the subsequent plan evaluation functions are displayed in a subsequent visual arrangement in a display. The iterative process continues until a treatment plan is selected.

Example #1

The iterative plan selection process is illustrated with the following example related to automatically or manually generated treatment plans. In the present example, four different first iteration treatment plans have been generated for the same patient. The four plans are each of a different treatment type. Further, in the present example, four different plan evaluation functions are determined for use in the first iteration.

The four different first iteration treatment plans in the present example are of the following types: (i) Plan 1: Optimised Gantry Angle Intensity Modulated Radiotherapy: (ii) Plan 2: Manually Set Gantry Angles Intensity Modulated Radiotherapy; (iii) Plan 3: Full Arc Volumetric Arc Therapy; and (iv) Plan 4: Butterfly Templatised Volumetric Arc Therapy.

In the present example, the four different types of first iteration plan evaluation functions are: (i) F_1: clinical goal based metrics; (ii) F_2: fluence complexity metrics; (iii) F_3: plan deliverability metrics; and (iv) F_4: plan accuracy metrics.

A person skilled in the art will be aware of the details of the above listed treatment plan types and plan evaluation function types, and so these need no elaboration here.

A weight is assigned to each of the first iteration plan evaluation functions. In the present example, the weights are denoted as a percentage, and plan evaluation function F_1 has a weight of 50%, plan evaluation function F_2 has a weight of 20%, plan evaluation function F_3 has a weight of 20% and plan evaluation function F_4 has a weight of 10%.

In the first iteration, each of the four different first iteration treatment plans may be evaluated using each of the four first iteration plan evaluation functions to provide four different costs for each of the four first iteration treatment plans. Each cost of each first iteration treatment plan may be weighted by the weight assigned to the respective first iteration plan evaluation function.

Each cost of each first iteration treatment plan may be normalised by the smallest cost for the respective first iteration treatment plan. The cost of a treatment plan as evaluated by plan evaluation function *n* may be written as:

$$C\_n$$

The minimum cost of a treatment plan as evaluated by all plan evaluation functions may be written as:

$$min(C\_n)$$

The normalised cost for a treatment plan as evaluated by plan evaluation function *n* may be written as:

$$C\_n/min(C\_n)$$

Further, each normalised cost for each treatment plan may be weighted by the weight assigned to the corresponding first iteration plan evaluation function.

Thus, the following metrics may be associated with each first iteration treatment plan: cost; weighted cost; normalised cost; and/or weighted normalised cost. Further, an overall "score" may be assigned to each treatment plan that may comprise any combination of one or more of: a sum of all the costs of the treatment plan; a sum of all the weighted costs of the treatment plan, a sum of all the normalised costs of the treatment plan, and a sum of all the weighted normalised costs of the treatment plan.

A first iteration visual arrangement is then created that shows the following information: an identity of each of the first iteration treatment plans, an identity of each of the first iteration plan evaluation functions, and one or more of: (i) each of the costs; (ii) each of the weighted costs, (iii) each of the normalised costs, (iv) each of the weighted normalised costs; and (v) the overall score; wherein (i)-(v) are the metrics associated with each first iteration treatment plan as described above.

That is, in the present example, the first iteration visual arrangement may show the following information:

Treatment plans evaluated:

Plan 1: Optimised Gantry Angle Intensity Modulated Radiotherapy
Plan 2: Manually Set Gantry Angles Intensity Modulated Radiotherapy
Plan 3: Full Arc Volumetric Arc Therapy
Plan 4: Butterfly Templatised Volumetric Arc Therapy

Plan evaluation functions used to evaluate the above treatment plans:

F_1: clinical goal based metrics
F_2: fluence complexity metrics
F_3: plan deliverability metrics
F_4: plan accuracy metrics

The first iteration visual arrangement may also show the following table of normalised costs and overall score associated with the treatment plans and the plan evaluation functions, wherein the overall score comprises a sum of weighted normalised costs.

Table 1: Example first iteration visual arrangement

|  | F_1 | F_2 | F_3 | F_4 | Overall score |
|---|---|---|---|---|---|
| **Weight** | 50% | 20% | 20% | 10% | |
| *Plan 1* | 3.0 | 2.0 | **1.0** | 1.5 | 2.25 |
| *Plan 2* | 1.1 | 1.5 | 2.1 | **1.0** | **1.37** |
| *Plan 3* | 2.0 | **1.0** | 2.2 | 2.4 | 1.88 |

(continued)

|  | F_1 | F_2 | F_3 | F_4 | Overall score |
|---|---|---|---|---|---|
| Weight | 50% | 20% | 20% | 10% |  |
| *Plan 4* | **1.0** | 1.2 | 2.5 | 2.2 | 1.46 |

The first visual arrangement is structured so that all the information in the first visual arrangement is comprehensible to a human user, for example in a single glance. The human user may then inspect the information in the first visual arrangement and make a balanced decision as to which treatment plan is most appropriate, given the metrics (one or more of the costs, weighted costs, normalised costs, weighted normalised costs and overall score) associated with each first iteration treatment plan.

By structuring the first visual arrangement so that all of this information is comprehensible to a human user at a single glance, the human user may make the following judgements (the following list is merely a non-limiting example):

"Plan 1 has the best plan deliverability metric, but the clinical goal based metric and the fluence complexity metric are not close enough to optimal (e.g., not close enough to 1.0)."

"Plan 4 has good quality clinical goal metrics and fluence complexity metrics, but has the worst plan deliverablity metric."

"Plan 3 obtains a good fluence complexity metric, but raises some doubt with respect to the clinical goal based metrics and the plan accuracy metrics."

"Comparing Plan 2 and Plan 3, Plan 2 is overall better and has the most balanced values for all the plan evaluation functions. Plan 2 is not the best in fluence complexity metrics, but from all other aspects it is better than Plan 3."

"The overall score gives additional numerical support for the conclusion that Plan 2 is the most appropriate, because Plan 2 has the least score. Nevertheless, the weighted score also shows that Plan 4 could be a candidate as its weighted score is not far from Plan 2."

If, by means of reasoning such as exemplified above, the user decides that a particular first iteration treatment plan's metrics are acceptable, the user then selects that particular treatment plan as the final selected plan. On the other hand, if the user decides that none of the first iteration treatment plans is suitable, then the user provides an input to proceed to a second iteration.

In the second iteration, in the present non-limiting example three second iteration treatment plans are generated for the same patient. The three plans may each be of a different treatment type, and may be different to the treatment types used in the first iteration.

Further, in the present example, three different plan evaluation functions are determined for use in the second iteration.

The three different second iteration treatment plans in the present example are: (i) Plan 1: Templatised Intensity Modulated Radiotherapy; (ii) Plan 2: Tangential Intensity Modulated Radiotherapy; (iii) Plan 3: Partial Arc Optimised Volumetric Arc Therapy.

In the present example, the three different types of second iteration plan evaluation functions are: (i) $F\_1$: fluence complexity metrics; (ii) $F\_2$: plan deliverability metrics; (iii) $F\_3$: plan accuracy metrics.

A weight is assigned to each of the second iteration plan evaluation functions. In the present example, the weights are denoted as a percentage, and plan evaluation function $F\_1$ has a weight of 40%, plan evaluation function $F\_2$ has a weight of 35%, and plan evaluation function $F\_3$ has a weight of 25%.

In the second iteration, each of the three different second iteration treatment plans may be evaluated using each of the three second iteration plan evaluation functions to provide three different costs for each of the three second iteration treatment plans. Each cost of each second iteration treatment plan may be weighted by the weight assigned to the respective second iteration plan evaluation function.

Each cost of each second iteration treatment plan may be normalised by the smallest cost for the respective second iteration treatment plan.

A second iteration visual arrangement is then created that shows the following information: an identity of each of the second iteration treatment plans, an identity of each of the second iteration plan evaluation functions, and one or more of: (i) each of the costs; (ii) each of the weighted costs, (iii) each of the normalised costs, (iv) each of the weighted normalised costs; and (v) the overall score; wherein (i)-(v) are metrics associated with each second iteration treatment plan, wherein the metrics are calculated in a similar fashion as explained above in relation to the first iteration.

That is, in the present example, the second iteration visual arrangement may show the following information:

Treatment plans evaluated:

Plan 1: Templatised Intensity Modulated Radiotherapy
Plan 2: Tangential Intensity Modulated Radiotherapy
Plan 3: Partial Arc Optimised Volumetric Arch Therapy

Plan evaluation functions used to evaluate the above treatment plans:

F_1: fluence complexity metrics
F_2: plan deliverability metrics
F_3: plan accuracy metrics

The second iteration visual arrangement may also show the following table of normalised costs and overall score associated with the treatment plans and the plan evaluation functions, wherein the overall score comprises a weighted sum of the normalised costs.

Table 2: Example second iteration visual arrangement

|  | F_1 | F_2 | F_3 | Overall score |
|---|---|---|---|---|
| **Weight** | 40% | 35% | 25% |  |
| *Plan 1* | 2.1 | **1.0** | 1.9 | **1.67** |
| *Plan 2* | **1.0** | 3.2 | 1.7 | 1.95 |
| *Plan 3* | 4.2 | 2.6 | **1.0** | 2.84 |

The second visual arrangement is structured so that all the information in the second visual arrangement is comprehensible to a human user, for example in a single glance. The human user may then inspect the information in the second visual arrangement and make a balanced decision as to which treatment plan is most appropriate, given the metrics (one or more of the costs, weighted costs, normalised costs, weighted normalised costs and overall score) associated with each second iteration treatment plan.

By structuring the second visual arrangement so that all of this information is comprehensible to a human user at a single glance, the human user may make the following judgements (the following list is a non-limiting example):

"Plan 3 has the worst fluence complexity metric, but performs well in plan accuracy."

"Plan 1 has the best plan deliverability metric, but has the worst plan accuracy and mediocre fluence complexity metric."

"Plan 2 excels in fluence complexity and has a second highest weighted score and so is an attractive option."

"Comparing Plan 1 and Plan 2, Plan 1 is overall better and has the most balanced values for all the plan evaluation functions. Plan 1, however, is worse than Plan 2 in terms of both fluence complexity and plan accuracy."

"The overall score gives quantitative support for the conclusion that Plan 1 is the most appropriate, because Plan 1 has the least overall score. Nevertheless, the overall score also shows that Plan 2 could be a candidate as its overall score is not far from Plan 1."

If the user decides that a particular second iteration treatment plan's metrics are acceptable, the user then selects that particular treatment plan as the final selected plan. On the other hand, if the user decides that none of the second iteration treatment plans is suitable, then the user provides an input to proceed to a third iteration.

The third and subsequent iterations may proceed in a similar way as described above in relation to the first and second iterations. The third and subsequent visual arrangements displayed in the third and subsequent iterations are similar to the first and second visual arrangements, but the information displayed in the third and subsequent visual arrangements use third iteration and subsequent iteration treatment plans and plan evaluation functions.

Each iteration ends with the display of a visual arrangement associated with that iteration. The iterative process stops when a human user, upon convenient inspection of a concise and informative visual arrangement, selects a treatment plan as the most appropriate treatment plan. The human user may use the concise information provided in the first, second, third or subsequent visual arrangements in making this selection.

The human user may also select a plurality of treatment plans at the end of the iterative process, and then re-start the iterative process using the plurality of selected treatment plans, for further comparison of the selected plurality of treatment plans.

Thus, by means of the present computer-guided iterative interactive process a human user is provided with assistance in searching for, evaluating and selecting a treatment plan.

In addition to assisting the human user in selecting a treatment plan, by showing all the information in the above-described visual arrangements in one glance to the planner, the planner may obtain important insights into whether the plans can be improved in a way that maintains the overall balanced quality in terms of all metrics.

In addition to varying the treatment plans and varying the plan evaluation functions from iteration to iteration, additionally, the weights of plan evaluation functions may also be varied from iteration to iteration.

A range of costs, weighted costs, normalised costs, weighted normalised costs, and/or overall scores may be determined corresponding to the varying weights, and optionally displayed in the first, second, third or subsequent visual arrangements.

A range of costs, weighted costs, normalised costs, weighted normalised costs, and/or overall scores may be determined corresponding to the varying plan evaluation functions, and optionally displayed in the first, second, third or subsequent visual arrangements.

A range of costs, weighted costs, normalised costs, weighted normalised costs, and/or overall scores may be determined corresponding to the varying treatment plans, and optionally displayed in the first, second, third or subsequent visual arrangements.

The computer-guided browsing, searching and evaluation of multiple treatment plans using multiple plan evaluation functions described above may be added to existing radiation treatment planning software or be provided as a novel software solution for radiation treatment planning.

Example #2

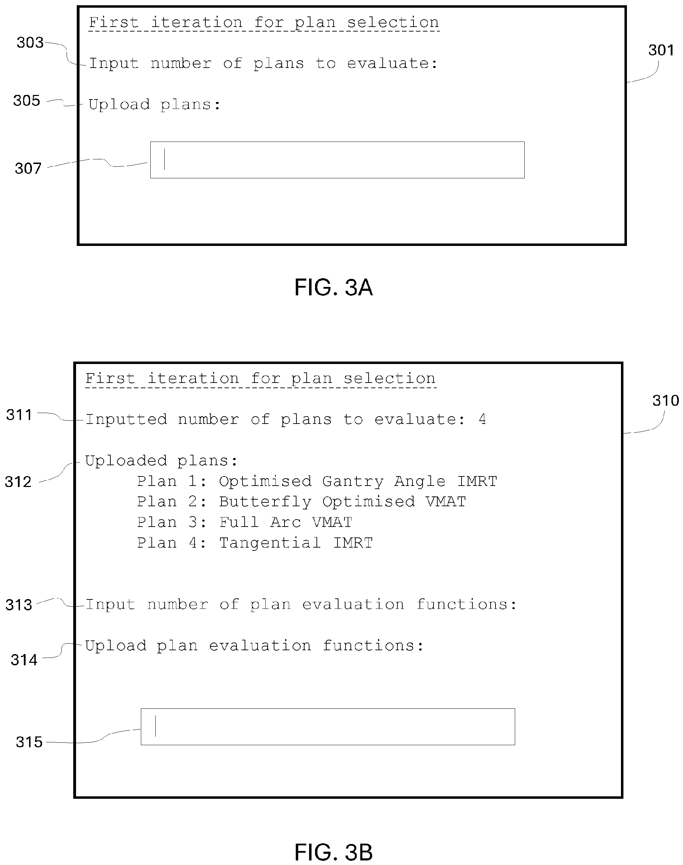

FIGS. 3A-3J show example screenshots of software implementing the present radiation treatment plan search and selection process. The treatment plan types and plan evaluation function types illustrated in this example are different from those presented in Example #1 above.

The iterative process of Example #2 is similar to that of Example #1. However, in Example #1 at the end of each iteration, the user is given the option of whether to change the treatment plans and/or whether to change the plan evaluation functions. In the present Example #2, the treatment plans are only inputted in the first iteration. In subsequent iterations, the same treatment plans are evaluated and there is no change in the treatment plans being evaluated. However, in the subsequent iterations, different plan evaluation functions are used to evaluate the same treatment plans.

Referring to FIG. 3A, a first iteration 301 begins the ongoing interactive and iterative plan evaluation and selection process. First a computer console provides a prompt 303 for the user to enter the number of treatment plans that are to be evaluated. The console also provides a prompt 305 for the user to upload the treatment plans. The user may enter the filenames associated with the treatment plans using input box 307. Alternatively or additionally, the user may upload the treatment plans using a drop-down menu.

Referring to FIG. 3B, a subsequent screenshot 310 of the first iteration is shown. The user has provided input 311 specifying that there are four plans to evaluate. The user has also uploaded four plans 312. The console then provides a prompt 313 for the user to enter the number of plan evaluation functions that are to be used to evaluate the treatment plans. The console also provides a prompt 314 for the user to upload the plan evaluation functions. The user may enter the filenames associated with the plan evaluation functions in box 315, or upload the plan evaluation functions using a drop-down menu. The drop-down menu may comprise a list of pre-existing plan evaluation functions.

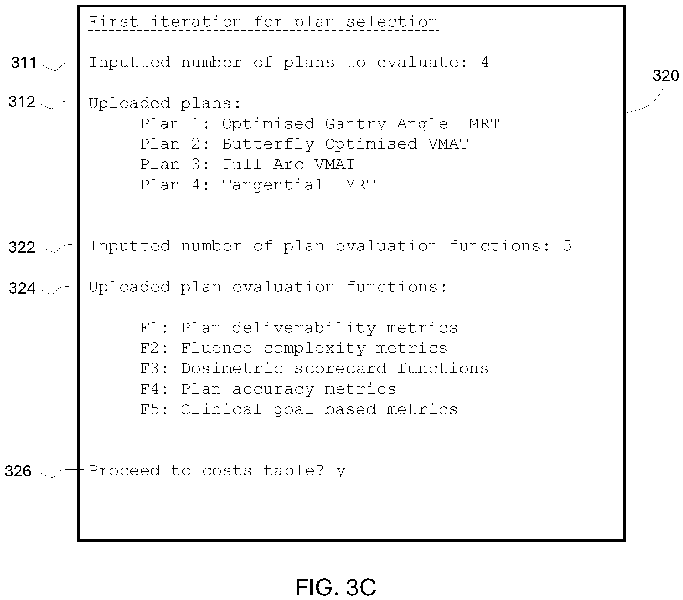

Referring to FIG. 3C, a subsequent screenshot 320 of the first iteration is shown. It can still be seen that the user has specified 311 that there are four plans to evaluate, and that the user has uploaded four plans 312. Additionally, in screenshot 320, the user has provided input 322 specifying that five plan evaluation functions are to be used in evaluating the treatment plans. The user has further uploaded the plan evaluation functions 324. Now that the user has specified: (i) a list 312 of treatment plans to be evaluated, and (ii) a list 324 of plan evaluation functions that are to be used to evaluate the treatment plans, the console provides a prompt 326 requesting the user to confirm that he wishes to proceed to display a table of costs. In the present example, the user has entered "y" in response to the prompt 326, indicating that he is ready to proceed to display the costs.

In response to receiving an input to proceed to display the costs, the processor applies each plan evaluation function to each radiation therapy treatment plan, to develop a plurality of costs for each treatment plan, as described above. Each cost for each treatment plan will reflect the cost of the respective treatment plan as determined by the respective plan evaluation function. Thus, in the present example, as there are four treatment plans and five plan evaluation functions, there will be five costs associated with each treatment plan, providing twenty costs in total.

The costs may be further processed by, for example, weighting each cost with a weight specific to the plan evaluation function used to determine the cost. The costs may also be normalised by dividing each cost with the minimum cost associated with the respective treatment plan. In addition to, or instead of, normalising the costs, the weighted costs may also be normalised by dividing each weighted cost associated with a treatment plan by the minimum weighted cost out of all the weighted costs associated with the treatment plan.

The costs table may display any combination of one or more of: costs, normalised costs, weighted costs and normalised weighted costs. The table may also display an overall score, which could be for example, a sum of all the costs, a sum of all the normalised costs, a sum of all the weighted costs and/or a sum of all the normalised weighted costs.

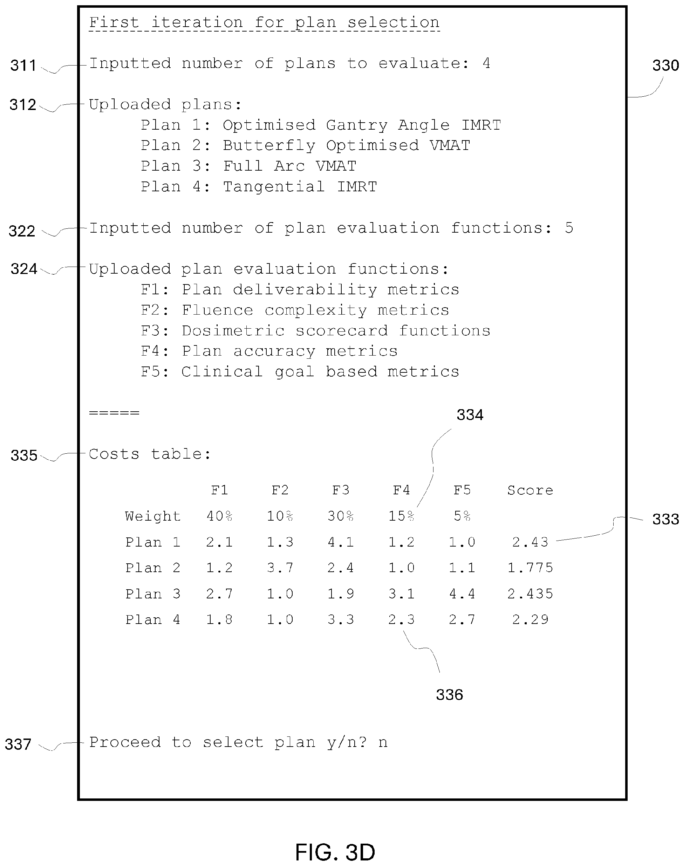

Referring to FIG. 3D, a subsequent screenshot 330 of the first iteration is shown. Reference numerals 311-324 refer to the same features as in FIG. 3C. The console now displays the normalised costs 336 associated with each treatment plan as calculated by the plurality of plan evaluation functions, in a costs table 335. In the present example, costs table 335 displays five normalised costs associated with each treatment plan as evaluated by five plan evaluation functions. Each plan evaluation function may have a corresponding weight 334. The normalised costs 336 for each treatment plan are weighted and summed to provide an overall score 333 for the treatment plan.

Thus, the visual arrangement or table 335 shows the following information in a concise manner: an identity of each treatment plan, an identity of each plan evaluation function, weights associated with each plan evaluation function, normalised costs associated with each treatment plan as determined by each plan evaluation function, and an overall score for each treatment plan comprising a weighted sum of the normalised costs. By providing this visual arrangement so that a human user may easily view all of the information in a single glance, the user is assisted in comparing the quality of each treatment plan as evaluated by the plurality of plan evaluation functions.

The above described first iteration 301 may be the first of a plurality of iterations that iteratively obtain treatment plan information and plan evaluation function information in an ongoing computer-guided search and selection process to find the most appropriate treatment plan for a particular patient. Thus, at the end of the first iteration, the computer console prompts 337 the user to input whether he is ready to select a treatment plan.

In the present example, the user is not satisfied with the costs associated with any of the treatment plans displayed in the costs table 335. For example, the user may not be satisfied at all with any of the plans and their associated costs. In other scenarios, the user may be satisfied with one of the plans in the costs table 335, but may wish to further assess this plan using further plan evaluation functions to be sure of his decision. The user may also be satisfied with one of the plans in the cost table 335 but may wish to double check that there are no other plans that may prove to be superior in future iterations.

Continuing with the present example, the user is not satisfied with any of the plans in cost table 335 and so the user provides an input "n" in response to the prompt 337, specifying that the user is not ready to select a treatment plan. In response to this input, the second iteration of the ongoing computer guided search and selection process to identify an appropriate treatment plan, commences.

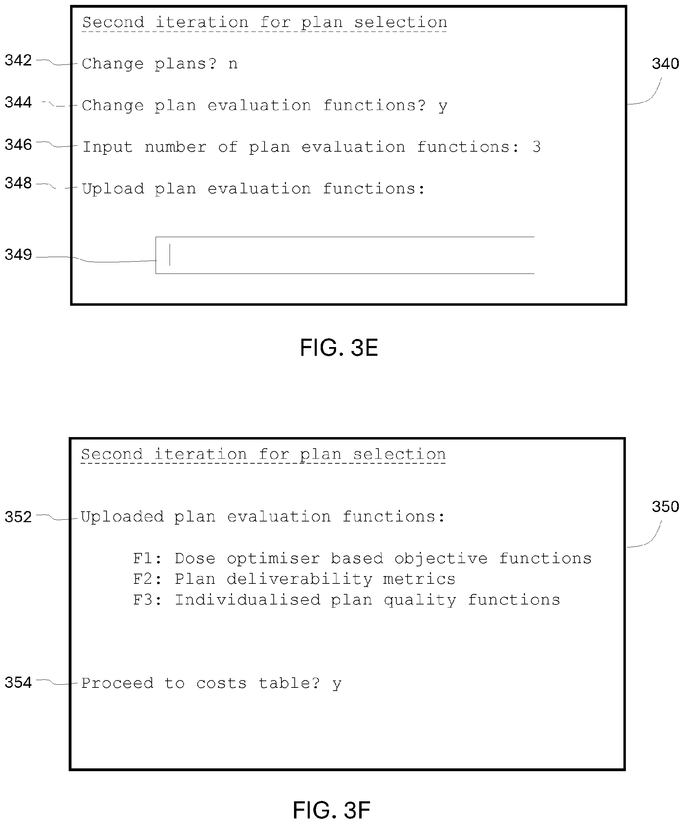

Referring to FIG. 3E, a second iteration 340 begins after the end of the first iteration 301. The console provides a prompt 342 for the user to specify whether the user wishes to change the treatment plans being evaluated. In the present example, the user does not wish to change the treatment plans being evaluated, and so has entered "n" ("No") in response to the prompt 342.

The console then provides a prompt 344 for the user to specify whether the user wishes to change the plan evaluation functions being used to evaluate the treatment plans. In the present example, the user does wish to change the plan evaluation functions, and so has entered "y" ("Yes") in response to the prompt 344.

The console then provides a prompt 346 for the user to specify how many plan evaluation functions are to be used to evaluate the plans of the second iteration. In the present example, the user has specified that there are three plan evaluation functions to be used in the second iteration.

The console then provides a prompt 348 to upload the plan evaluation functions, using the input box 349. The plan evaluation functions may be uploaded by means of a drop-down menu comprising, for example, a list of pre-existing evaluation functions.

Referring to FIG. 3F, a subsequent screenshot 350 of the second iteration is shown. It can be seen that the user has entered three plan evaluation functions 352. Now that the user has specified the list of treatment plans to be evaluated in the second iteration (by inputting that there is to be no change in the treatment plans being evaluated in the first iteration) and has specified the list of plan evaluation functions to be used in the second iteration, the console prompts 354 the user to confirm that the user wishes to proceed to display the costs associated with the plans, as determined by the plan evaluation functions.

In response to receiving an input to proceed to display the costs of the second iteration, the processor applies each plan evaluation function of the second iteration to each radiation therapy treatment plan of the second iteration, to develop a plurality of costs for each treatment plan. Each cost for each treatment plan will reflect the cost of the respective treatment plan as determined by the respective plan evaluation function. Thus, in the present example, as there are four treatment plans and three plan evaluation functions, there will be three costs associated with each treatment plan, providing twelve costs in total.

As for the first iteration, in the second iteration the costs may be further processed by, for example, weighting each cost with a weight specific to the plan evaluation function used to determine the cost. Also, as in the first iteration, in the second iteration the costs may be normalised by dividing each cost with a minimum cost associated with a respective treatment plan. Also, the weighted costs themselves may be normalised by dividing each weighted cost associated with a treatment plan by the minimum weighted cost out of all the weighted costs associated with the treatment plan. As in the first iteration, the costs table of the second iteration may display any combination of one or more of: costs, normalised costs, weighted costs and normalised weighted costs. As in the first iteration, the table of the second iteration may also display an overall score, which could be for example, a sum of all the costs, a sum of all the normalised costs, a sum of all the weighted costs and/or a sum of all the normalised weighted costs.

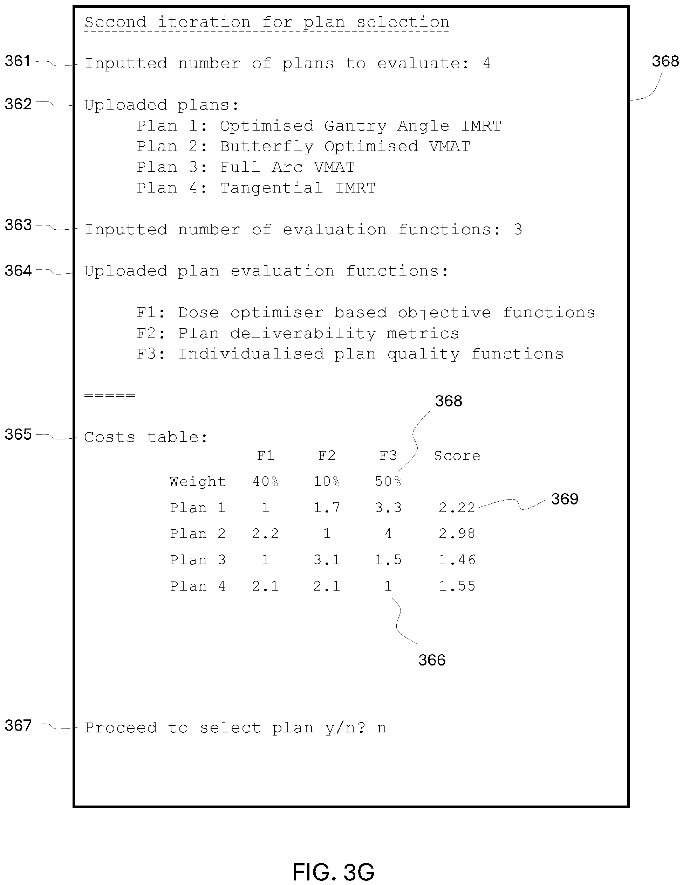

Referring to FIG. 3G, reference numerals 361-364 refer to similar features as reference numerals 311-324 of FIG. 3C, except that the information displayed is that of the second iteration. The console now displays the normalised costs 366 associated with each treatment plan as calculated by the plan evaluation functions, in a costs table 365. In the present example, costs table 365 displays three normalised costs associated with each treatment plan as evaluated by three plan evaluation functions. Each plan evaluation function may have a corresponding weight 368. The normalised costs 366 for each treatment plan are weighted and summed to provide an overall score 369 for the respective treatment plan.

Thus, as in the first iteration, the visual arrangement or table 365 of the second iteration shows the following information in a concise manner: an identity of each treatment plan of the second iteration, an identity of each plan evaluation function of the second iteration, weights associated with each plan evaluation function of the second iteration, normalised costs associated with each treatment plan as determined by each plan evaluation function, and an overall score for each treatment plan of the second iteration comprising a weighted sum of the normalised costs. By providing this visual arrangement so that a human user may easily view all of the information in a single glance, the user is assisted in comparing the quality of each treatment plan of the second iteration as evaluated by the plurality of plan evaluation functions of the second iteration.

The above described second iteration 340 may be the second of a plurality of iterations that iteratively obtain treatment plan information and plan evaluation function information in an ongoing computer-guided search and selection process to find the most appropriate treatment plan for a particular patient. Thus, at the end of the second iteration, the computer console prompts 367 the user to input whether he is ready to select a treatment plan. In the present example, the user is still not satisfied with the costs associated with any of the treatment plans displayed in the costs table. Thus, the user provides an input "n" to the prompt 367, specifying that the user is not ready to select a treatment plan. In response to this input, the method proceeds to the third iteration of the ongoing computer guided search and selection process to identify an appropriate treatment plan.

The third and subsequent iterations proceed in a similar way to the first and second iterations described above.

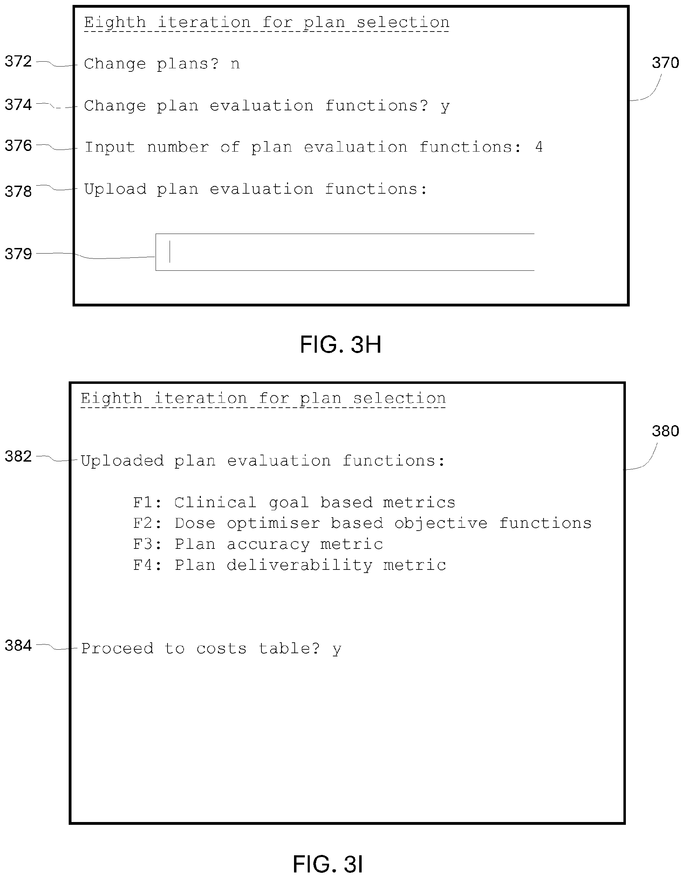

The iterative process stops when the user provides an input specifying that he is ready to select a plan from the displayed costs table. In the present example, the user enters such an input at the eighth iteration. This is merely an example, and in practice there may be more or fewer iterations. Although the first iteration 301, the second iteration 340 and the eighth iteration 370 are shown in FIGS. 3A-3D, 3E-3G and 3H-3J respectively, the third, fourth, fifth, sixth and seventh iterations are not shown here for brevity and clarity.

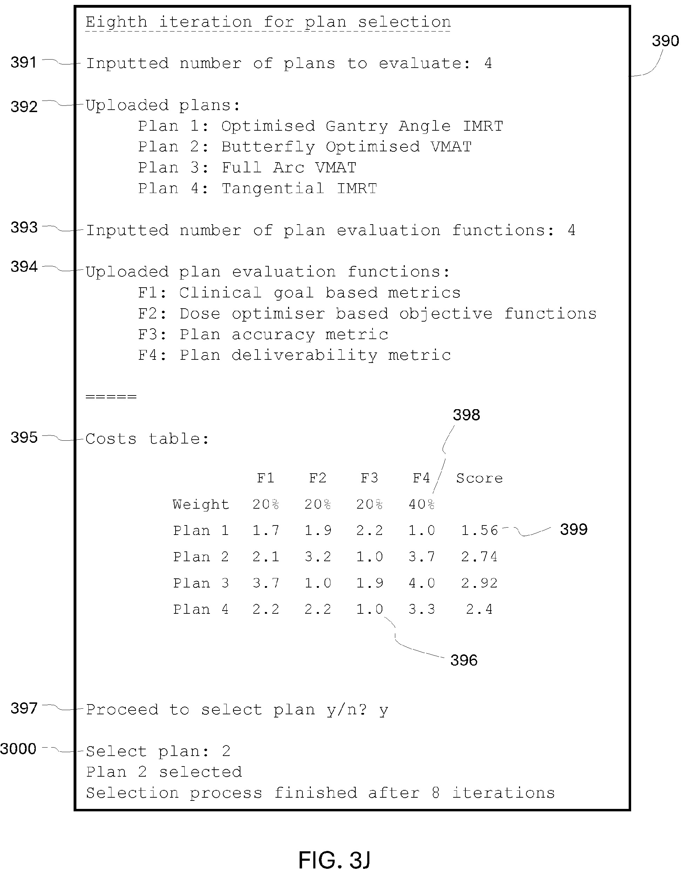

Referring to FIG. 3H-3J, the eighth iteration 370 proceeds in a similar manner to the second iteration described in relation to FIGS. 3E-3G. That is, as shown in FIG. 3H, the user is prompted 372 to input whether he wishes to change the treatment plans and specifies "n" (i.e., "no") in reply; the user is then prompted 374 whether he wishes to change the plan evaluation functions and specifies "y" (i.e., "yes") in reply; the user is then prompted 376 to input the number of plan evaluation functions and in this case has entered that there will be four plan evaluation functions to be used in the eighth iteration. The user is then prompted 378 to upload the plan evaluation functions. The user is enabled to upload the plan evaluation functions using the input box 379, or a drop-down menu. The drop-down menu may comprise a list of pre-existing plan evaluation functions. Next, as shown in the screenshot 380 of FIG. 3I, the user uploads four plan evaluation functions 382, and then specifies in response to prompt 384 that he wishes to proceed to display the costs table. In the screenshot 390 of FIG. 3J, the console displays the number of plans 391, the plans 392, the number of plan evaluation functions 393 and the plan evaluation functions 394. The costs table 395 is displayed in a similar way as described in relation to costs table 335 and costs table 365.

Thus, as in the first iteration, the second iteration and the subsequent iterations, the visual arrangement or table 395 of the eighth iteration shows the following information in a concise manner: an identity of each treatment plan of the eighth iteration, an identity of each plan evaluation function of the eighth iteration, weights 398 associated with each plan evaluation function of the eighth iteration, normalised costs 396 associated with each treatment plan as determined by each plan evaluation function, and an overall score 399 for each treatment plan of the eighth iteration comprising a weighted sum of the respective normalised costs. By providing this visual arrangement so that a human user may easily view all of the information in a single glance, the user is assisted in comparing the quality of each treatment plan of the eighth iteration as evaluated by the plurality of plan evaluation functions of the eighth iteration.

At the end of the eighth iteration, the computer console prompts 397 the user to input whether he is ready to select a treatment plan. In the present example, the user inputs that he is ready to select a plan. That is, by means of the concise and compact visual arrangement providing all the required information at a single glance, the user determines, at reference numeral 3000, that plan 2 of the eighth iteration is the best plan out of all the other plans that have been analysed. That is, in the present example, the user determines that "Butterfly Optimised VMAT" is the most appropriate treatment plan for the particular patient.

Thus, by means of a computer guided search and selection process, the user is able to identify and select the most appropriate treatment plan for the patient. The user may then proceed to carry out the selected treatment plan on the patient.

Overall method

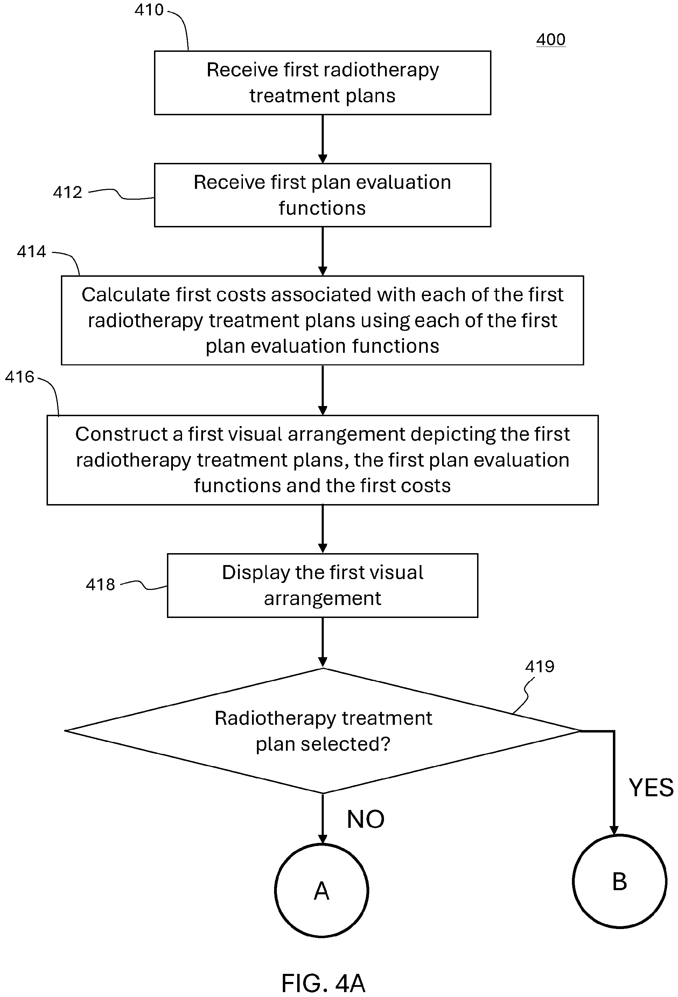

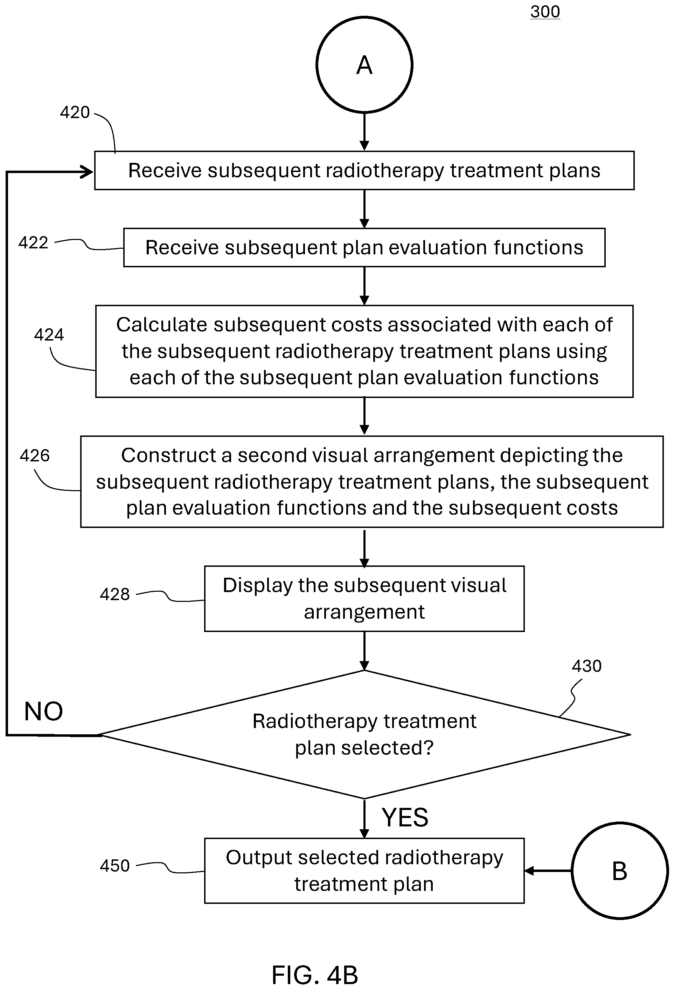

FIGS. 4A and 4B show a flowchart of a method that uses multiple plan evaluation functions in the evaluation and selection of a radiation treatment plan.

In step 410, the radiotherapy treatment plan apparatus receives a plurality of first radiotherapy treatment plans.

In step 412, the radiotherapy treatment plan apparatus receives a plurality of first plan evaluation functions.

In step 414, the radiotherapy treatment plan apparatus calculates first costs associated with each of the first radiotherapy treatment plans using each of the first plan evaluation functions.

In step 416, the radiotherapy treatment plan apparatus constructs a first visual arrangement showing an identity of each of the first radiotherapy treatment plans, an identity of each of the first plan evaluation functions, and the first costs associated with the first radiotherapy treatment plans.

In step 418, the radiotherapy treatment plan apparatus displays the first visual arrangement for user inspection.

In step 419, the radiotherapy treatment plan apparatus provides a prompt to the user to specify if the user is ready to select a plan. If the user provides an affirmative response, the method proceeds to step 450 where the user selected plan is outputted. If the user provides a negative response, the method proceeds to step 420.

In step 420, the radiotherapy treatment plan apparatus may receive a plurality of subsequent radiotherapy treatment plans. This step is optional, and the subsequent radiotherapy treatment plans may be the same as the first radiotherapy treatment plans, that is, no new radiotherapy treatment plans may be received at step 420. Alternatively, the subsequent radiotherapy treatment plans may comprise new radiotherapy treatment plans.

In step 422, the radiotherapy treatment plan apparatus may receive a plurality of subsequent plan evaluation functions.

In step 424, the radiotherapy treatment plan apparatus may calculate subsequent costs associated with each of the subsequent radiotherapy treatment plans using each of the subsequent plan evaluation functions.

In step 426, the radiotherapy treatment plan apparatus may construct a subsequent visual arrangement showing an identity of each of the subsequent radiotherapy treatment plans, an identify of each of the subsequent plan evaluation functions, and the subsequent costs.

In step 428, the radiotherapy treatment plan apparatus may display the subsequent visual arrangement for user inspection.

In step 430, the radiotherapy treatment plan apparatus provides a prompt to the user to specify if the user is ready to select a plan. If the user provides an affirmative response, the method proceeds to step 450 where the user selected plan is outputted. If the user provides a negative response, the method proceeds to step 420, where a third or further iterations are performed similar to the first and subsequent iterations described above.

The iterative process stops when the user selects a plan at either step 419 or step 430 and the selected plan is outputted in step 450.

At the end of each iteration, a visual arrangement or costs table is displayed comprising (i) the identity of treatment plans; (ii) the identity of plan evaluation functions; and (iii) one or more of: costs, weighted costs, normalised costs, normalised weighted costs, and/or overall score, associated with each treatment plan. By showing this visual arrangement in a concise manner in a display that can be viewed by a user in a single glance, the user is assisted in the selection of a radiotherapy treatment plan by means of an ongoing computer guided interactive and iterative search and selection process.

As described above, a range of weighted costs may be calculated by using varying weights for each of the plan evaluation functions. A range of costs may be calculated corresponding to the different plan evaluation functions. A range of costs may be calculated corresponding to the different radiotherapy treatment plans. One or more of these ranges may be included in the corresponding visual arrangement.

Plan evaluation function types

The types of plan evaluation functions that may be used to evaluate treatment plans in the present computer guided process for the search and selection of plans may be of any type.

Some non-limiting examples are listed below:

> *Plan quality functions of automated optimisers*
> *Dosimetric scorecard functions*
> *Clinical goal based metrics*
> *Fluence complexity metrics*
> *Plan deliverability metrics*
> *Plan accuracy metrics*
> *Dose optimiser based objective functions*

A skilled person will be aware of the above listed types of plan evaluation functions, and further elaboration is not required here.

*Individualised plan quality functions:* In this (novel) type of plan evaluation function, a dosimetrist (or oncologist) may specify narrowed-down and focused quality criteria for reaching a subset of dosimetric goals for personalised treatment of a given patient. In other words, a dosimetrist may choose a focused subset of goals he wants to reach and is particularly interested in. For example, in head and neck treatments, this individualised plan quality function could focus on and measure the dose to parotid and submandibular glands, aiming to get as small a dose to them as possible.

Treatment plan types

The treatment plans that may be evaluated in the present computer guided process for the search and selection of plans may relate to radiotherapy treatment modalities of any type.

Some non-limiting examples are listed below:

> *Intensity Modulated Radiotherapy*
> *Templatised Intensity Modulated Radiotherapy*
> *Tangential Intensity Modulated Radiotherapy*
> *Optimised Gantry Angle Intensity Modulated Radiotherapy*
> *Manually Set Gantry Angles Intensity Modulated Radiotherapy*
> *Volumetric Arc Therapy*
> *Full Arc Volumetric Arc Therapy*
> *Butterfly Templatised Volumetric Arc Therapy*
> *Butterfly Optimised Volumetric Arc Therapy*
> *Butterfly Manually Set Volumetric Arc Therapy*
> *Partial Arc Templatised Volumetric Arc Therapy*
> *Partial Arc Optimised Volumetric Arc Therapy*
> *Partial Arc Manually Set Volumetric Arc Therapy*

A skilled person will be aware of the above listed types of radiotherapy treatment modalities, and further elaboration is not required here.

Computer system

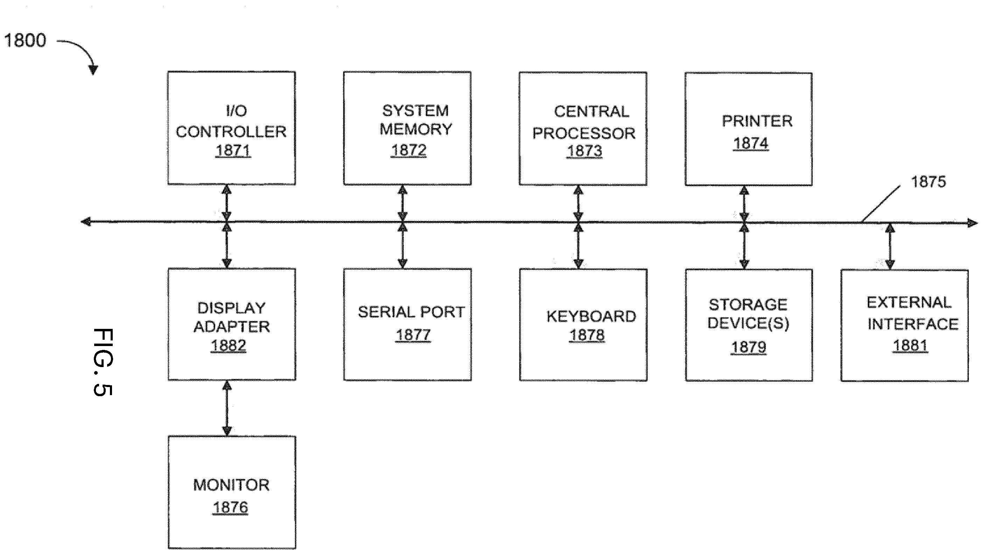

Any of the methods mentioned herein may utilise a computer system or any suitable number of computer subsystems. Examples of such subsystems are shown in FIG. 5 in computer system 1800. In some embodiments, a computer system includes a single computer apparatus, where the subsystems can be the components of the computer apparatus. In other embodiments, a computer system can include multiple computer apparatuses, each being a subsystem, with internal components.

The subsystems shown in FIG. 5 are interconnected via a system bus 1875. Additional subsystems such as a printer 1874, keyboard 1878, storage device(s) 1879, monitor 1876, which is coupled to display adapter 1882, and others are shown. Peripherals and input/output (I/O) devices, which couple to I/O controller 1871, can be connected to the computer system by any number of means known in the art, such as serial port 1877. For example, serial port 1877 or external interface 1881 (e.g. Ethernet, Wi-Fi, etc.) can be used to connect computer system 1800 to a wide area network such as the Internet, a mouse input device, or a scanner. The interconnection via system bus 1875 allows the central processor 1873 to communicate with each subsystem and to control the execution of instructions from system memory 1872 or the storage device(s) 1879 (e.g., a fixed disk, such as a hard drive or optical disk), as well as the exchange of information between subsystems. The system memory 1872 and/or the storage device(s) 1879 may embody a computer readable medium. Any of the data mentioned herein can be output from one component to another component and can be output to the user.

A computer system can include a plurality of the same components or subsystems, e.g., connected together by external interface 1881 or by an internal interface. In some embodiments, computer systems, subsystems, or apparatuses can communicate over a network. In such instances, one computer can be considered a client and another computer a server, where each can be part of a same computer system. A client and a server can each include multiple systems, subsystems, or components.

It should be understood that any of the embodiments of the present invention can be implemented in the form of control logic using hardware (e.g. an application specific integrated circuit or field programmable gate array) and/or using computer software with a generally programmable processor in a modular or integrated manner. As used herein, a processor includes a multi-core processor on a same integrated chip, or multiple processing units on a single circuit board. Based on the disclosure and teachings provided herein, a person of ordinary skill in the art will know and appreciate other ways and/or methods to implement embodiments of the present invention using hardware and a combination of hardware and software.

Any of the computer implemented methods described in this application may be implemented as software code to be executed by a processor using any suitable computer language such as, for example, Java, C++ or Perl using, for example, conventional or object-oriented techniques. The software code may be stored as a series of instructions or commands on a computer readable medium for storage and/or transmission; suitable media include random access memory (RAM), a read only memory (ROM), a magnetic medium such as a hard-drive or a floppy disk, or an optical medium such as a compact disk (CD) or DVD (digital versatile disk), flash memory, and the like. The computer readable medium may be any combination of such storage or transmission devices.

Such programs may also be encoded and transmitted using carrier signals adapted for transmission via wired, optical, and/or wireless networks conforming to a variety of protocols, including the Internet. As such, a computer readable medium according to an embodiment of the present invention may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps of the methods. Thus, embodiments can be directed to computer systems configured to perform the steps of any of the methods described herein, potentially with different components performing a respective step or a respective group of steps. Although presented as numbered steps, steps of methods herein can be performed at a same time or in a different order. Additionally, portions of these steps may be used with portions of other steps from other methods. Also, all or portions of a step may be optional. Additionally, any of the steps of any of the methods can be performed with modules, circuits, or other means for performing these steps.

Those skilled in the art will recognise that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A radiotherapy treatment plan generation apparatus for searching for and selecting a radiotherapy treatment plan by an iterative process, the apparatus configured to receive one or more first radiotherapy treatment plans and further configured to:

   in a first iteration:

      receive one or more first plan evaluation functions;
      calculate first costs associated with each of the first radiotherapy treatment plans using each of the first plan evaluation functions;
      construct a first visual arrangement depicting an identity of each of said first radiotherapy treatment plans, an identity of each of said first plan evaluation functions and first costs; and
      display said first visual arrangement;

and in one or more subsequent iterations:

receive one or more subsequent plan evaluation functions;
calculate subsequent costs associated with each of the first radiotherapy treatment plans using each of the subsequent plan evaluation functions;
construct a subsequent visual arrangement depicting an identity of each of said first radiotherapy treatment plans, an identity of each of said subsequent plan evaluation functions and subsequent costs;
display said subsequent visual arrangement;
stop the iterative process upon receiving a selection of a final radiotherapy treatment plan; and
outputting said selected final radiotherapy treatment plan.

2. The apparatus of claim 1, wherein the first plan evaluation functions and/or the subsequent plan evaluation functions comprise two or more plan evaluation functions that relate to different types of plan evaluation functions, wherein said types of plan evaluation functions include two or more of:

(i) plan quality functions of automated optimisers;
(ii) dosimetric scorecard functions;
(iii) clinical goal based metrics;
(iv) fluence complexity metrics;
(v) plan deliverability metrics;
(vi) plan accuracy metrics;
(vii) dose optimiser based objective functions; and
(viii) individualised plan quality functions.

3. The apparatus of claim 1 or claim 2, wherein the first plan evaluation functions and/or the subsequent plan evaluation functions comprise different plan evaluation functions of the same type.

4. The apparatus of any preceding claim, wherein the first radiotherapy treatment plans comprise two or more radiotherapy treatment plans that relate to different types of radiotherapy, wherein said types of radiotherapy include two or more of:

(i) Templatised Intensity Modulated Radiotherapy;
(ii) Tangential Intensity Modulated Radiotherapy;
(iii) Optimised Gantry Angle Intensity Modulated Radiotherapy;
(iv) Manually Set Gantry Angles Intensity Modulated Radiotherapy;
(v) Full Arc Volumetric Arc Therapy;
(vi) Butterfly Templatised Volumetric Arc Therapy;
(vii) Butterfly Optimised Volumetric Arc Therapy;
(viii) Butterfly Manually Set Volumetric Arc Therapy;
(ix) Partial Arc Templatised Volumetric Arc Therapy;
(x) Partial Arc Optimised Volumetric Arc Therapy; and
(xi) Partial Arc Manually Set Volumetric Arc Therapy.

5. The apparatus of any preceding claim, wherein the first radiotherapy treatment plans comprise different radiotherapy treatment plans of the same type.

6. The apparatus of any preceding claim, further configured to weight one or more of the first and/or subsequent costs using respective weights associated with each respective first and/or subsequent plan evaluation function.

7. The apparatus of any preceding claim, further configured to determine a plurality of normalised costs for each of the first radiotherapy treatment plans by dividing each of the first and/or subsequent costs of each first radiotherapy treatment plans by a minimum cost out of all the costs for the respective first radiotherapy treatment plan; and optionally:
wherein the apparatus is further configured to weight one or more of the plurality of normalised costs using respective weights associated with each respective first and/or subsequent plan evaluation function.

8. The apparatus of any preceding claim, further configured to vary weights associated with the first and/or subsequent plan evaluation functions and re-calculate the weighted costs and/or weighted normalised costs of the first radiotherapy treatment plans using the varying weights.

9. The apparatus of any preceding claim, further configured to calculate, and optionally display, a range of costs corresponding to varying weights associated with the first and/or subsequent plan evaluation functions.

10. The apparatus of any preceding claim, further configured to calculate, and optionally display, a range of costs corresponding to the different first and/or subsequent plan evaluation functions.

11. The apparatus of any preceding claim, further configured to calculate, and optionally display, a range of costs corresponding to the different radiotherapy treatment plans.

12. The apparatus of any preceding claim, further configured to calculate, and optionally display, a total score for each of the first radiotherapy treatment plans comprising one or more of: a sum of costs, a sum of weighted costs, a sum of normalised costs and a sum of weighted normalised costs.

13. The apparatus of any preceding claim further configured to include in the first visual arrangement or in the subsequent visual arrangement, one or more of:

   (i) weighted first and/or subsequent costs for the first radiotherapy treatment plans,
   (ii) normalised first and/or subsequent costs for the first radiotherapy treatment plans,
   (iii) weighted normalised first and/or subsequent costs for the first radiotherapy treatment plans, and
   (iv) a total score for the first radiotherapy treatment plans,

   as determined using each of the first and/or subsequent plan quality functions and corresponding weights.

14. The apparatus of any preceding claim further configured to, in the one or more subsequent iterations:

   receive one or more subsequent radiotherapy treatment plans;
   calculate subsequent costs associated with each of the subsequent radiotherapy treatment plans using each of the first and/or subsequent plan evaluation functions;
   and
   construct the subsequent visual arrangement depicting said subsequent radiotherapy treatment plans, first and/or subsequent plan evaluation functions and subsequent costs.

15. The apparatus of any preceding claim, further configured to select the final radiotherapy treatment plan wholly or partially by a processor;
   and optionally:
   wherein the processor is configured to select the final radiotherapy treatment plan in dependence on one or more of: (i) respective costs, (ii) respective weighted costs, (iii) respective normalised costs, (iv) respective weighted normalised costs, and (v) respective total score.

FIG. 1

FIG. 2

First iteration for plan selection

303 — Input number of plans to evaluate:

305 — Upload plans:

307 —

301

**FIG. 3A**

First iteration for plan selection

311 — Inputted number of plans to evaluate: 4

312 — Uploaded plans:
        Plan 1: Optimised Gantry Angle IMRT
        Plan 2: Butterfly Optimised VMAT
        Plan 3: Full Arc VMAT
        Plan 4: Tangential IMRT

313 — Input number of plan evaluation functions:

314 — Upload plan evaluation functions:

315 —

310

**FIG. 3B**

```
First iteration for plan selection
------------------------------------

Inputted number of plans to evaluate: 4

Uploaded plans:
        Plan 1: Optimised Gantry Angle IMRT
        Plan 2: Butterfly Optimised VMAT
        Plan 3: Full Arc VMAT
        Plan 4: Tangential IMRT



Inputted number of plan evaluation functions: 5

Uploaded plan evaluation functions:

        F1: Plan deliverability metrics
        F2: Fluence complexity metrics
        F3: Dosimetric scorecard functions
        F4: Plan accuracy metrics
        F5: Clinical goal based metrics



Proceed to costs table? y
```

FIG. 3C

First iteration for plan selection

Inputted number of plans to evaluate: 4

Uploaded plans:
      Plan 1: Optimised Gantry Angle IMRT
      Plan 2: Butterfly Optimised VMAT
      Plan 3: Full Arc VMAT
      Plan 4: Tangential IMRT

Inputted number of plan evaluation functions: 5

Uploaded plan evaluation functions:
      F1: Plan deliverability metrics
      F2: Fluence complexity metrics
      F3: Dosimetric scorecard functions
      F4: Plan accuracy metrics
      F5: Clinical goal based metrics

=====

Costs table:

|        | F1   | F2   | F3   | F4   | F5  | Score |
|--------|------|------|------|------|-----|-------|
| Weight | 40%  | 10%  | 30%  | 15%  | 5%  |       |
| Plan 1 | 2.1  | 1.3  | 4.1  | 1.2  | 1.0 | 2.43  |
| Plan 2 | 1.2  | 3.7  | 2.4  | 1.0  | 1.1 | 1.775 |
| Plan 3 | 2.7  | 1.0  | 1.9  | 3.1  | 4.4 | 2.435 |
| Plan 4 | 1.8  | 1.0  | 3.3  | 2.3  | 2.7 | 2.29  |

Proceed to select plan y/n? n

FIG. 3D

Second iteration for plan selection

342 — Change plans? n

344 — Change plan evaluation functions? y

346 — Input number of plan evaluation functions: 3

348 — Upload plan evaluation functions:

349 —

340

## FIG. 3E

Second iteration for plan selection

352 — Uploaded plan evaluation functions:

    F1: Dose optimiser based objective functions
    F2: Plan deliverability metrics
    F3: Individualised plan quality functions

354 — Proceed to costs table? y

350

## FIG. 3F

Second iteration for plan selection

Inputted number of plans to evaluate: 4

Uploaded plans:
    Plan 1: Optimised Gantry Angle IMRT
    Plan 2: Butterfly Optimised VMAT
    Plan 3: Full Arc VMAT
    Plan 4: Tangential IMRT

Inputted number of evaluation functions: 3

Uploaded plan evaluation functions:

    F1: Dose optimiser based objective functions
    F2: Plan deliverability metrics
    F3: Individualised plan quality functions

=====

Costs table:

| | F1 | F2 | F3 | Score |
|---|---|---|---|---|
| Weight | 40% | 10% | 50% | |
| Plan 1 | 1 | 1.7 | 3.3 | 2.22 |
| Plan 2 | 2.2 | 1 | 4 | 2.98 |
| Plan 3 | 1 | 3.1 | 1.5 | 1.46 |
| Plan 4 | 2.1 | 2.1 | 1 | 1.55 |

Proceed to select plan y/n? n

FIG. 3G

Eighth iteration for plan selection

Change plans? n

Change plan evaluation functions? y

Input number of plan evaluation functions: 4

Upload plan evaluation functions:

370

## FIG. 3H

Eighth iteration for plan selection

Uploaded plan evaluation functions:

    F1: Clinical goal based metrics
    F2: Dose optimiser based objective functions
    F3: Plan accuracy metric
    F4: Plan deliverability metric

Proceed to costs table? y

380

## FIG. 3I

```
Eighth iteration for plan selection

Inputted number of plans to evaluate: 4

Uploaded plans:
      Plan 1: Optimised Gantry Angle IMRT
      Plan 2: Butterfly Optimised VMAT
      Plan 3: Full Arc VMAT
      Plan 4: Tangential IMRT

Inputted number of plan evaluation functions: 4

Uploaded plan evaluation functions:
      F1: Clinical goal based metrics
      F2: Dose optimiser based objective functions
      F3: Plan accuracy metric
      F4: Plan deliverability metric


=====

Costs table:

                F1    F2    F3    F4    Score
        Weight  20%   20%   20%   40%
        Plan 1  1.7   1.9   2.2   1.0   1.56
        Plan 2  2.1   3.2   1.0   3.7   2.74
        Plan 3  3.7   1.0   1.9   4.0   2.92
        Plan 4  2.2   2.2   1.0   3.3   2.4



Proceed to select plan y/n? y

Select plan: 2
Plan 2 selected
Selection process finished after 8 iterations
```

FIG. 3J

410

400

Receive first radiotherapy
treatment plans

412

Receive first plan evaluation
functions

414

Calculate first costs associated with each of the first
radiotherapy treatment plans using each of the first
plan evaluation functions

416

Construct a first visual arrangement depicting the first
radiotherapy treatment plans, the first plan evaluation
functions and the first costs

418

Display the first visual
arrangement

419

Radiotherapy treatment
plan selected?

NO

YES

A

B

FIG. 4A

300

A

420 Receive subsequent radiotherapy treatment plans

422 Receive subsequent plan evaluation functions

424 Calculate subsequent costs associated with each of the subsequent radiotherapy treatment plans using each of the subsequent plan evaluation functions

426 Construct a second visual arrangement depicting the subsequent radiotherapy treatment plans, the subsequent plan evaluation functions and the subsequent costs

428 Display the subsequent visual arrangement

430 Radiotherapy treatment plan selected?

NO

YES

450 Output selected radiotherapy treatment plan

B

FIG. 4B

FIG. 5

Europäisches
Patentamt
European
Patent Office
Office européen
des brevets

# EUROPEAN SEARCH REPORT

**Application Number**

EP 24 20 8070

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/299469 A1 (HARJU ARI [FI] ET AL) 30 September 2021 (2021-09-30) * figures 4-6, 8 * * paragraphs [0055] - [0060] * * paragraphs [0067] - [0069] * * paragraphs [0074] - [0077] * ----- | 1-15 | INV. A61N5/10 |
| X | MEYER ET AL: "A Multiplan Treatment-Planning Framework: A Paradigm Shift for Intensity-Modulated Radiotherapy", INTERNATIONAL JOURNAL OF RADIATION: ONCOLOGY BIOLOGY PHYSICS, PERGAMON PRESS, USA, vol. 68, no. 4, 13 July 2007 (2007-07-13), pages 1178-1189, XP022153391, ISSN: 0360-3016, DOI: 10.1016/J.IJROBP.2007.02.051 * page 1180 - page 1183 * * figures 5,6 * * table 3 * * sections: "Plan ranking scheme", "Composite criteria" and "Pre-emptive selection" * ----- | 1-15 | |
| X | EP 4 176 926 A1 (KONINKLIJKE PHILIPS NV [NL]) 10 May 2023 (2023-05-10) * figure 3 * * paragraphs [0054] - [0065] * ----- | 1,2,4,5 | TECHNICAL FIELDS SEARCHED (IPC) A61N |
| A | US 2021/213303 A1 (BOKRANTZ RASMUS [SE] ET AL) 15 July 2021 (2021-07-15) * figures 1a, 1b * * paragraph [0076] * ----- | 1-15 | |

The present search report has been drawn up for all claims

| Place of search | Date of completion of the search | Examiner |
|---|---|---|
| Munich | 14 March 2025 | Seiferle, Benedict |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 8070

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2025

| Patent document cited in search report | | Publication date | Patent family member(s) | | Publication date |
|---|---|---|---|---|---|
| US 2021299469 | A1 | 30-09-2021 | NONE | | |
| EP 4176926 | A1 | 10-05-2023 | CN | 118234542 A | 21-06-2024 |
| | | | EP | 4176926 A1 | 10-05-2023 |
| | | | EP | 4429760 A1 | 18-09-2024 |
| | | | US | 2025025719 A1 | 23-01-2025 |
| | | | WO | 2023083637 A1 | 19-05-2023 |
| US 2021213303 | A1 | 15-07-2021 | CN | 112203722 A | 08-01-2021 |
| | | | EP | 3581241 A1 | 18-12-2019 |
| | | | JP | 7508378 B2 | 01-07-2024 |
| | | | JP | 2021527468 A | 14-10-2021 |
| | | | US | 2021213303 A1 | 15-07-2021 |
| | | | WO | 2019238602 A1 | 19-12-2019 |

EPO FORM P0459

For more details about this annex : see Official Journal of the European Patent Office, No. 12/82